# EUROPEAN PATENT APPLICATION

(11) **EP 1 880 990 A1**
(43) Date of publication of application: **23.01.2008**
(21) Application number: 06745496.7
(22) Date of filing: 20.04.2006
(51) Int. Cl.: C07C 211/60

(54) **NOVEL ORGANIC ELECTROLUMINESCENT MATERIAL, ORGANIC ELECTROLUMINESCENT ELEMENT EMPLOYING THE SAME, AND THIN-FILM-FORMING SOLUTION FOR ORGANIC ELECTROLUMINESCENCE**

(30) Priority: 09.05.2005 JP 2005136573; 26.10.2005 JP 2005311774
(71) Applicant: IDEMITSU KOSAN CO., LTD., Tokyo 100-8321 (JP)
(72) Inventor: INOUE, Tetsuya, 2990293 (JP); KONDO, Hirofumi, 2990293 (JP); JUNKE, Tadanori, 2990293 (JP)
(74) Representative: Gille Hrabal Struck Neidlein Prop Roos
(86) International application number: PCT/JP2006/308315
(87) International publication number: WO 2006/120859

(57) **Abstract**

An aromatic amine compound having a specific structure. An organic electroluminescence device which is composed of one or more organic thin film layers sandwiched between a pair of electrodes consisting of an anode and a cathode, wherein at least one of the organic thin film layers contains the aromatic amine compound. An organic electroluminescence device rendering various kinds of light emission hue, having high heat resistance, with a prolonged lifetime, an enhanced luminance of light emission and an enhanced efficiency of light emission, together with a novel aromatic amine compound and a thin-film-forming solution for an organic electroluminescence device with high solubility and with film-forming ability by wet process all realizing the organic electroluminescence device are provided.

## Description

### TECHNICAL FIELD

The present invention relates to an aromatic amine compound and an organic electroluminescence device employing the same. Particularly, it relates to an organic electroluminescence device rendering various kinds of light emission hue, having high heat resistance, with a prolonged lifetime, an enhanced luminance of light emission and an enhanced efficiency of light emission; to a novel aromatic amine compound with high solubility and with film-forming ability by wet process; and to a thin-film-forming solution for an organic electroluminescence device realizing the organic electroluminescence device.

### BACKGROUND ART

Organic electroluminescence (EL) devices are used for a planar light emitting member such as a flat panel display of wall televisions and a back light of displays, and the development of EL devices has been widely conducted. Electroluminescenct phenomenon of an organic substance was observed about an anthracene single crystal by Pope in 1963 (Non-patent Document 1), and Helfrich and Schneider were successful in observation of comparatively strong injection-type EL by using a solution electrode-based substance with favorable injection efficiency in 1965 (Non-patent Document 2). As reported since then, a study of forming an organic photoluminescent substance employing a conjugate organic host substance and a conjugate organic activating agent having a fused benzene ring was investigated and naphthalene, anthracene, phenanthrene, tetracene, pyrene, benzopyrene, chrysene, picene, carbazole, fluorene, biphenyl, terphenyl, triphenylene oxide, dihalobiphenyl, trans-stilbene, 1,4-diphenyl butadiene and so on were described as examples of the organic host substance. Further, anthracene, tetracene, pentacene and so on were described as examples of the activating agent. However, those organic photoluminescent substances each exists as single layer having thickness of exceeding 1 µm, and a high electric field was necessary for light emission. Accordingly, studies about a thin film device in accordance with a vacuum vapor deposition process were advanced (e.g. Non-patent Document 3). However, although the thin film technology was effective for reduction of driving voltage, achieving practical use level devices with high luminance of light emission was not successful. Tang et al. devised an organic EL device laminating two extremely thin films (a hole transporting layer and a light emitting layer) in accordance with a vacuum vapor deposition process between an anode and a cathode thereby realized high luminance of light emission with low driving voltage (Non-patent Document 4 or Patent Document 1). Afterwards, as a result of advancing development of an organic compound employable for the hole transporting layer and the light emitting layer for ten and several years, lifetime and efficiency of light emission both with practical use level are achieved. Resultantly, practical use of the organic EL device is started from display units of car stereos, portable telephones, etc.
However, because the luminance of light emission and durability of time lapse after a long time usage are not sufficient in a viewpoint of practical use, further improvement is demanded.

As means for solving the problems, oligomer (trimer, tetramer) amine is used in order to improve glass transition temperature (Tg) of a hole injecting and transporting material. For example, Patent Document 2 discloses a compound represented by the following general formula (A): 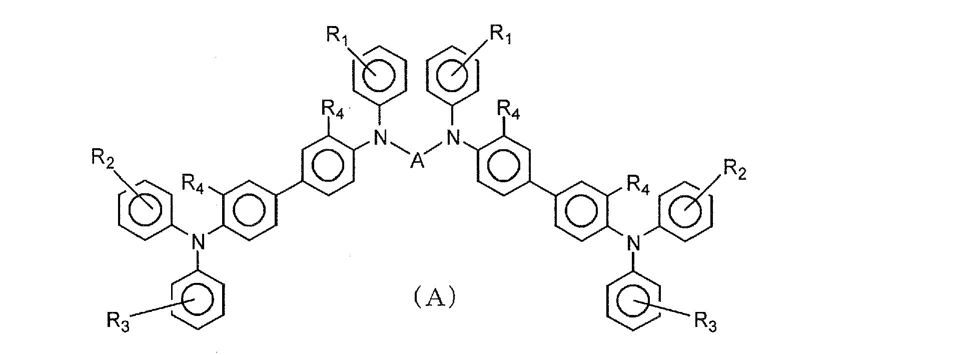 (In the formula, R₁, R₂ and R₃ may be the same with or different from each other, and each represents a hydrogen atom, a lower alkyl group or a lower alkoxy group; R₄ represents a hydrogen atom, a lower alkyl group, a lower alkoxy group or a chlorine atom; A represents: 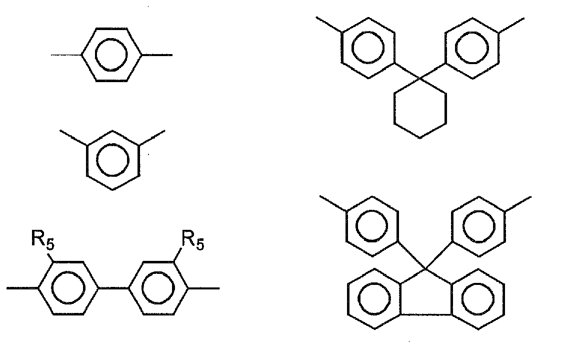 wherein R₅ represents a hydrogen atom, a lower alkyl group, a lower alkoxy group or a chlorine atom.)

Patent Document 3 discloses a compound represented by the following general formula (B):

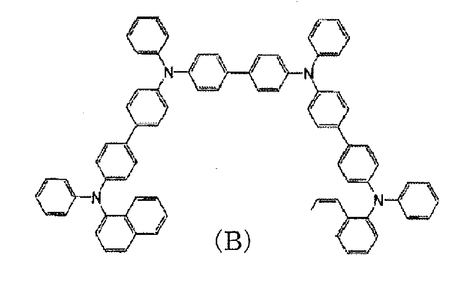

Patent Document 4 discloses a compound represented by the following general formula (C): 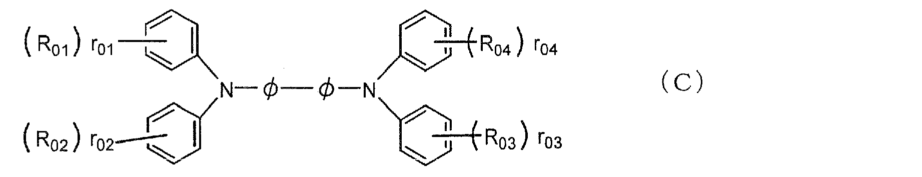 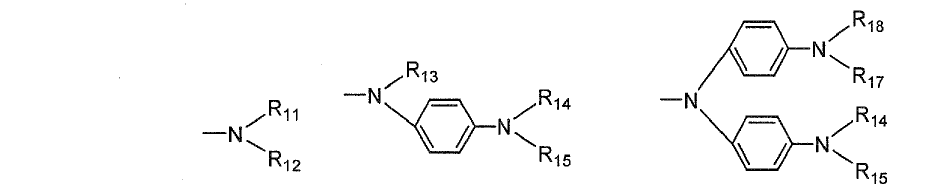 wherein φ represents a phenylene group, R₀₁, R₀₂, R₀₃, and R₀₄ each independently represents a diarylaminophenylene group, r₀₁, r₀₂, r₀₃ and r₀₄ each represents an integer of 0 to 5, and r₀₁+r₀₂+r₀₃+r₀₄ represents 1 or more; R₁₁, R₁₂, R₁₃, R₁₄, R₁₅, R₁₆ and R₁₇ each independently represents a substituted or unsubstituted aryl group respectively.

Patent Document 5 discloses a compound represented by the following general formula (D): 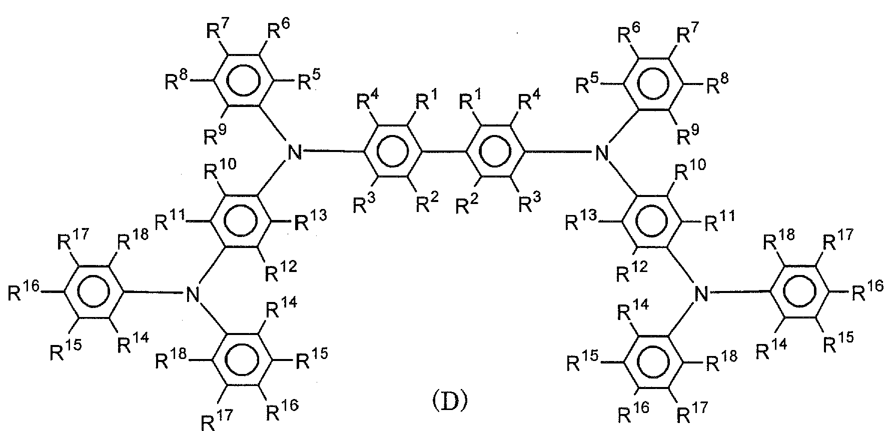 (In the formula, R₁ to R₁₈ each represents a group independently selected from a group consisting of a hydrogen atom, a lower alkyl group and a lower alkoxy group respectively.)

Further, Patent Document 6 discloses a compound represented by the following general formula (E): 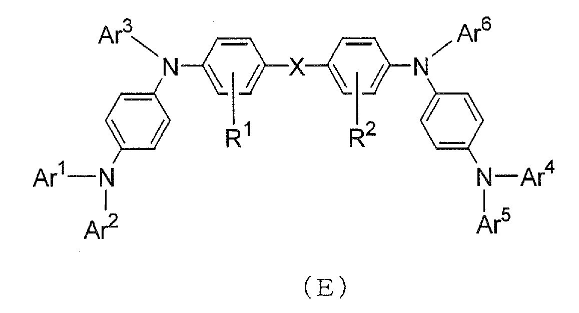 (Ar¹ to Ar⁶ each independently represents a hydrogen atom, an alkyl group or an alkoxy group each having 1 to 6 carbon atoms, an aryl group having 6 to 24 ring carbon atoms or an aryl group having 6 to 24 ring carbon atoms which may be substituted by a styryl group. X represents a linking group, which represents a single bond, an arylene group having 6 to 24 ring carbon atoms, an alkylene group having 1 to 6 carbon atoms, diphenylmethylene, an ether bond, a thioether bond, a substituted or unsubstituted vinyl bond or an aromatic heterocycle. R¹ and R² each independently represents an alkyl group having 1 to 6 carbon atoms, an alkoxy group or a hydrogen atom, which may be bonded each other to form a substituted or unsubstituted five-membered ring or six-membered ring.)
However, the compounds described in Patent Documents 2 to 6 did not achieve sufficient hole injecting properties.

Furthermore, as means for improving the hole injecting property, a compound made by introducing a fluorenyl group has been used. For example, Patent Document 7 discloses a luminescent device employing a compound represented by the following general formula (F) as a hole transporting material. 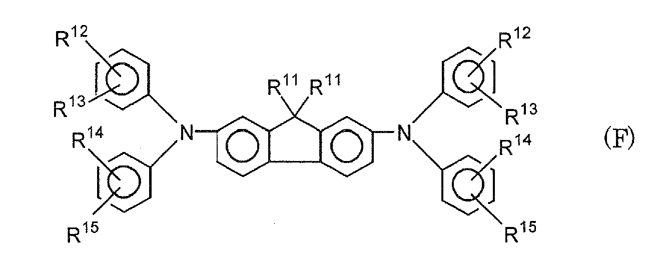 (In the formula, R¹¹ represents an alkyl group or an aralkyl group, R¹², R¹³, R¹⁴ and R¹⁵ each independently represents a hydrogen atom, an alkyl group, an alkoxy group or a halogen atom.)
Because Tg of the compound was 100 °C or smaller, and the device employing the compound had short lifetime and a poor heat resistance never achieving practical use.

Patent Document 8 discloses a luminescent device employing a compound represented by the following general formula (G) obtained by improving the general formula (F) as a hole transporting material. 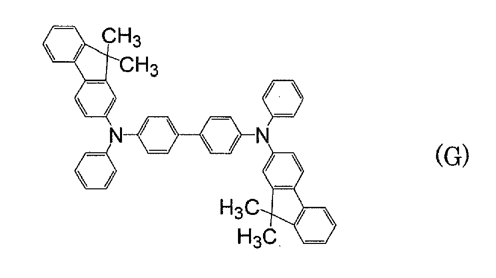 Although an enhancement of glass transition temperature and an enhancement of the hole injecting property are recognized about the compound, there was a problem that it still had short lifetime.

Further, Patent Document 9 discloses a compound represented by the following general formula (H): 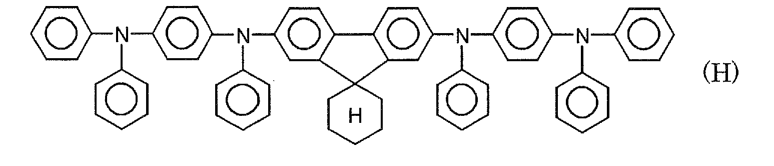 Despite an enhancement of the hole injecting property recognized about the compound, a further enhancement of glass transition temperature and an extension of lifetime were demanded.

Patent Document 1: U. S. Pat. No. 4,356,429
Patent Document 2: Japanese Patent No. 3220950
Patent Document 3: JP 2000-86595A
Patent Document 4: JP 2000-156290A
Patent Document 5: JP 9-301934A
Patent Document 6: JP 2000-309566A
Patent Document 7: JP 5-25473A
Patent Document 8: JP 11-35532A
Patent Document 9: JP 2000-80433A
Non-patent Document 1: J. Chem. Phys., 38 (1963) 2042
Non-patent Document 2: Phys. Rev. Lett., 14 (1965) 229
Non-patent Document 3: Thin Solid Films, 94 (1982) 171
Non-patent Document 4: Appl. Phys. Lett., 51 (1987) 913

### DISCLOSURE OF THE INVENTION

### PROBLEMS TO BE SOLVED BY THE INVENTION

In order for solving the above problems, the present invention has objects of providing an aromatic amine compound and an organic electroluminescence device employing the compound. Particularly, the present invention has objects of providing an organic electroluminescence device rendering various kinds of light emission hue, having high heat resistance, with a prolonged lifetime, an enhanced luminance of light emission and an enhanced efficiency of light emission; providing a novel aromatic amine compound with high solubility and with film-forming ability by wet process and a thin-film-forming solution for an organic electroluminescence device both realizing the organic electroluminescence device.

### MEANS FOR SOLVING THE PROBLEM

As a result of intensive researches and studies to achieve the above objects by the present inventors, it was found that an employment of an aromatic amine compound represented by the following general formula (1) overcomes the above problems, resultantly completing the present invention.
Namely, the present invention provides an aromatic amine compound represented by the following general formula (1):

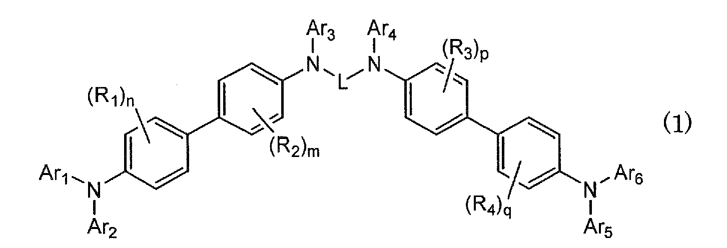

In the general formula (1), Ar₁ to Ar₆ each independently represents a substituted or unsubstituted aryl group having 6 to 40 ring carbon atoms or a substituted or unsubstituted heteroaryl group having 3 to 40 ring carbon atoms. Additionally, each pair of Ar₁ and Ar₂, and Ar₅ and Ar₆ may be bonded each other with single bond, and may form a carbazolyl group. R₁ to R₄ each independently represents a halogen atom, a carboxyl group, an amino group, a hydroxy group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, or a substituted or unsubstituted alkoxy group having 1 to 40 carbon atoms. n, m, p and q each represents an integer of 0 to 4 respectively.
L is a divalent linking group represented by the following general formula (2) or the following general formula (3).

-(Ar₇)ₐ-(Ar₈)_{b}-(Ar₉)_{c}- (2)

In the general formula (2), Ar₇ to Ar₉ are the same as described above about Ar₁ to Ar₆, a, b and c each represents an integer of 1 to 3. 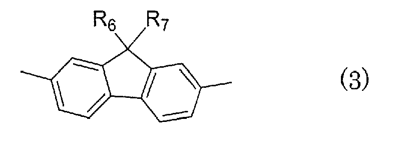 In the general formula (3), R₆ and R₇ are the same as described above about R₁ to R₄, and a pair of R₆ and R₇ may be bonded each other to form a ring structure.

Further, the present invention provides an organic EL device which is composed of one or more organic thin film layers including at least one light emitting layer and sandwiched between a cathode and an anode, wherein at least one of the organic thin film layers contains the aromatic amine compound singly or as a component of a mixture. The organic EL device of the present invention reveals various kinds of light emission hue, a high heat resistance, a long lifetime, an enhanced luminance of light emission and an enhanced efficiency of light emission.
Furthermore, the present invention provides a novel aromatic amine compound having high solubility and film-forming ability by wet process, and also provides a thin-film-forming solution for an organic electroluminescence device all in order for realizing the above function.

### EFFECT OF THE INVENTION

The aromatic amine compound of the present invention has high solubility, and a laminated coating of the thin-film-forming solution using the compound in a production process of the organic EL device is possible, exhibiting film-forming ability by wet process. The organic EL device using the compound reveals various kinds of light emission hue, and a high heat resistance. Particularly, employing the aromatic amine compound of the present invention as a hole injecting and transporting material will provide the device with an enhanced luminance of light emission, an enhanced efficiency of light emission and a prolonged lifetime because of its improved hole injecting and transporting property.

### PREFERRED EMBODIMENT TO CARRY OUT THE INVENTION

The present invention provides an aromatic amine compound represented by the following general formula (1): 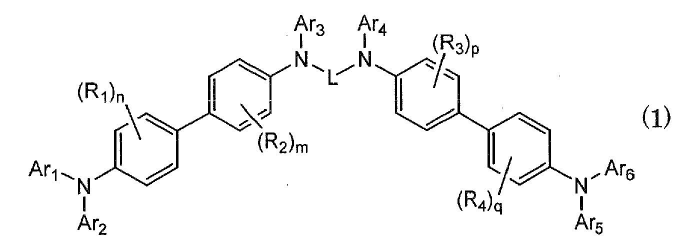 In the general formula (1), Ar₁ to Ar₆ each independently represents a substituted or unsubstituted aryl group having 6 to 40 ring carbon atoms or a substituted or unsubstituted heteroaryl group having 3 to 40 ring carbon atoms. Additionally, each pair of Ar₁ and Ar₂, and Ar₅ and Ar₆ may be bonded each other with single bond, and may form a carbazolyl group. R₁ to R₄ each independently represents a halogen atom, a carboxyl group, an amino group, a hydroxy group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, or a substituted or unsubstituted alkoxy group having 1 to 40 carbon atoms, n, m, p and q each represents an integer of 0 to 4.

In the general formula (1), examples of the aryl group having 6 to 40 ring carbon atoms represented by Ar₁ to Ar₆ include phenyl group, 2-biphenylyl group, 3-biphenylyl group, 4-biphenylyl group, terphenyl group, 3,5-diphenylphenyl group, 3,5-di(1-naphthyl)phenyl group, 3,5-di(2-naphthyl)phenyl group, 3,4-diphenylphenyl group, pentaphenylphenyl group, 4-(2,2-diphenylvinyl)phenyl group, 4-(1,2,2-triphenylvinyl)phenyl group, fluorenyl group, 1-naphthyl group, 2-naphthyl group, 4-(1-naphthyl)phenyl group, 4-(2-naphthyl)phenyl group, 3-(1-naphthyl)phenyl group, 3-(2-naphthyl)phenyl group, 9-anthryl group, 2-anthryl group, 9-phenanthryl group, 1-pyrenyl group, crycenyl group, naphthacenyl group, coronyl group, etc.

In the general formula (1), examples of heteroaryl group having 3 to 40 ring carbon atoms represented by Ar₁ to Ar₆ include 1-pyrrolyl group, 2-pyrrolyl group, 3-pyrrolyl group, pyrazinyl group, pyrimidyl group, pyridazyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthroline-2-yl group, 1,7-phenanthroline-3-yl group, 1,7-phenanthroline-4-yl group, 1,7-phenanthroline-5-yl group, 1,7-phenanthroline-6-yl group, 1,7-phenanthroline-8-yl group, 1,7-phenanthroline-9-yl group, 1,7-phenanthroline-10-yl group, 1,8-phenanthroline-2-yl group, 1,8-phenanthroline-3-yl group, 1,8-phenanthroline-4-yl group, 1,8-phenanthroline-5-yl group, 1, 8 -phenanthroline -6-yl group, 1,8-phenanthroline-7-yl group, 1,8-phenanthroline-9-yl group, 1,8-phenanthroline-10-yl group, 1,9-phenanthroline-2-yl group, 1,9-phenanthroline-3-yl group, 1,9-phenanthroline-4-yl group, 1,9-phenanthroline-5-yl group, 1,9-phenanthroline-6-yl group, 1,9-phenanthroline-7-yl group, 1,9-phenanthroline-8-yl group, 1,9-phenanthroline-10-yl group, 1,10-phenanthroline-2-yl group, 1,10-phenanthroline-3-yl group, 1, 10-phenanthroline-4-yl group, 1,10-phenanthroline-5-yl group, 2,9-phenanthroline-1-yl group, 2,9-phenanthroline-3-yl group, 2,9-phenanthroline-4-yl group, 2,9-phenanthroline-5-yl group, 2,9-phenanthroline-6-yl group, 2,9-phenanthroline-7-yl group, 2,9-phenanthroline-8-yl group, 2,9-phenanthroline-10-yl group, 2,8-phenanthroline-1-yl group, 2,8-phenanthroline-3-yl group, 2,8-phenanthroline-4-yl group, 2,8-phenanthroline-5-yl group, 2,8-phenax2throline-6-yl group, 2,8-phenanthroline-7-yl group, 2,8-phenanthroline-9-yl group, 2,8-phenanthroline-10-yl group, 2,7-phenanthroline-1-yl group, 2,7-phenanthroline-3-yl group, 2,7-phenanthroline-4-yl group, 2,7-phenanthroline-5-yl group, 2,7-phenanthroline-6-yl group, 2,7-phenanthroline-8-yl group, 2,7-phenanthroline-9-yl group, 2,7-phenanthroline-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrole-1-yl group, 2-methylpyrrole-3-yl group, 2-methylpyrrole-4-yl group, 2-methylpyrrole-5-yl group, 3-methylpyrrole-1-yl group, 3-methylpyrrole-2-yl group, 3-methylpyrrole-4-yl group, 3-methylpyrrole-5-yl group, 2-t-butylpyrrole-4-yl group, 3-(2-phenylpropyl)pyrrole-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, etc.

In the general formula (1), examples of the halogen atom represented by the substituents R₁ to R₄ include fluorine atom, chlorine atom, bromine atom, iodine atom and astatine atom.

In the general formula (1), examples of the substituted or unsubstituted alkyl group having 1 to 50 carbon atoms represented by the substituents R₁ to R₄ include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloro isobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-trliodopropyl group, aminomethyl group, 1-aminoethyl group, 2-aminoethyl group, 2-amino isobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamantyl group, 2-adamantyl group, 1-norbornyl group, 2-norbornyl group, etc.

In the general formula (1), examples of the substituted or unsubstituted alkenyl group having 2 to 40 carbon atoms represented by the substituents R₁ to R₄ include vinyl group, allyl group, 1-butenyl group, 2-butenyl group, 3-butenyl group, 1,3-butanedienyl group, 1-methylvinyl group, styryl group, 2,2-diphenylvinyl group, 1,2-diphenylvinyl group, 1-methylallyl group, 1,1-dimethylallyl function, 2-methylallyl group, 1-phenylallyl group, 2-phenylallyl group, 3-phenylallyl group, 3,3-diphenylallyl group, 1,2-dimethylallyl group, 1-phenyl-l-butenyl group, 3-phenyl-1-butenyl group, etc.

In the general formula (1), examples of the substituted or unsubstituted alkynyl group having 2 to 40 carbon atoms represented by the substituents R₁ to R₄ include ethynyl group, propargyl group, 3-pentynyl group, etc.

In the general formula (1), the substituted or unsubstituted alkoxy group having 1 to 50 carbon atoms represented by the substituents R₁ to R₄ is a group represented by -OY₁, and examples of Y₁ include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, etc.

In the general formula (1), L is a divalent linking group represented by the following general formula (2) or the following general formula (3).

-(Ar₇)ₐ-(Ar₈)_{b}-(Ar₉)_{c},- (2)

In the general formula (2), Ar₇ to Ar₉ are the same as described above about Ar₁ to Ar₆, a, b and c each represents an integer of 1 to 3. 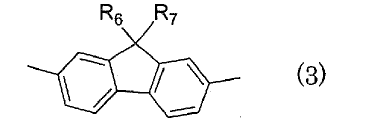 In the general formula (3), R₆ and R₇ are the same as described above about R₁ to R₄, and a pair of R₆ and R₇ may be bonded each other to form a ring structure.

Specific examples of the aromatic amine compound of the present invention represented by the general formula (1) include the following compounds, though not limited thereto.

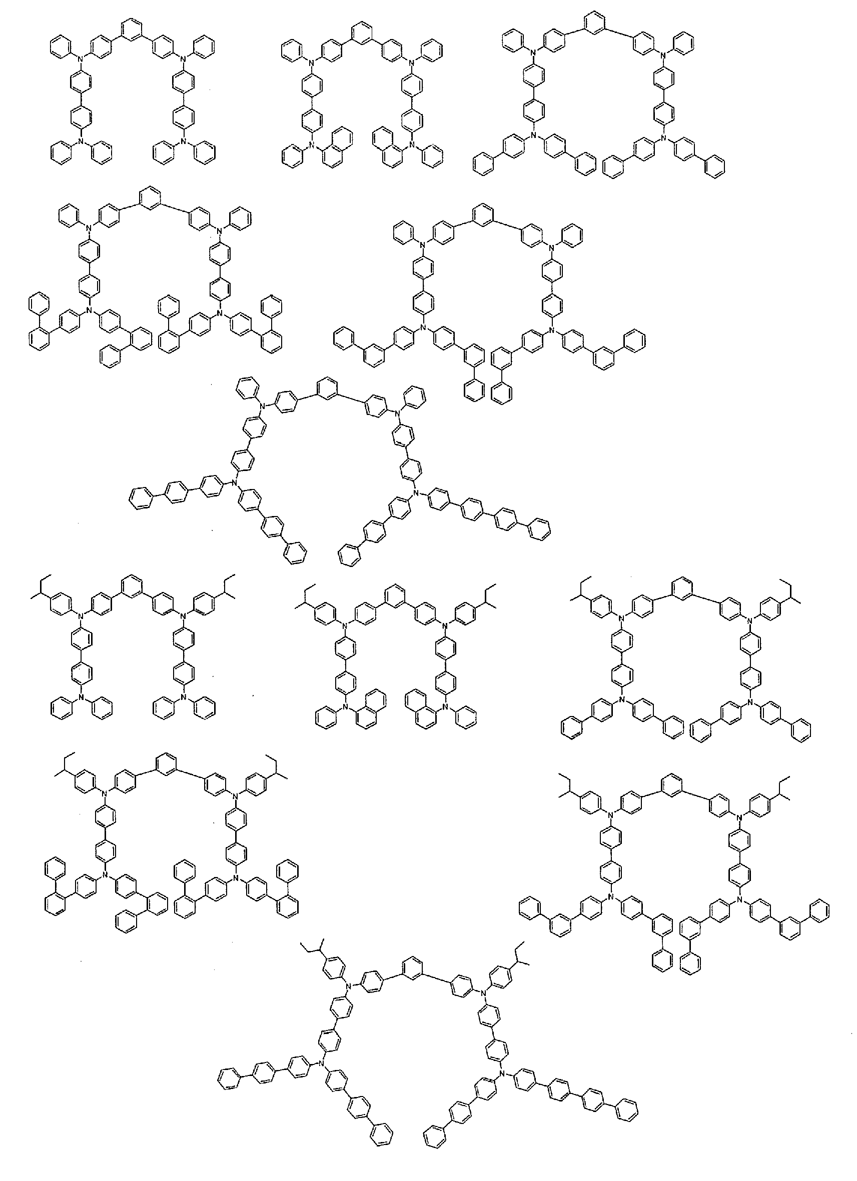

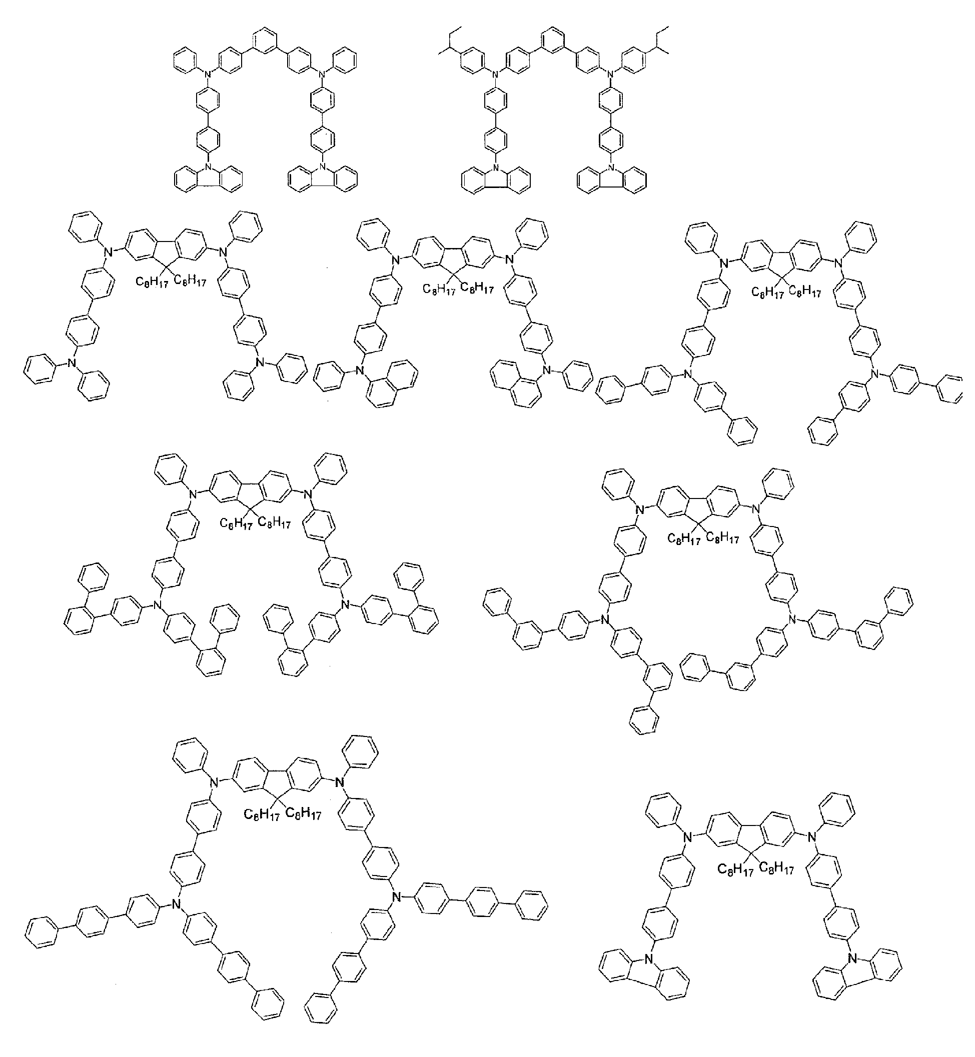

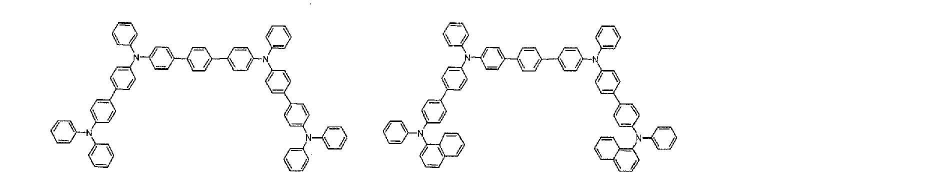

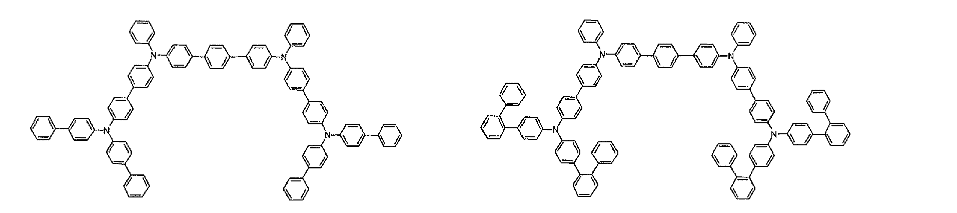

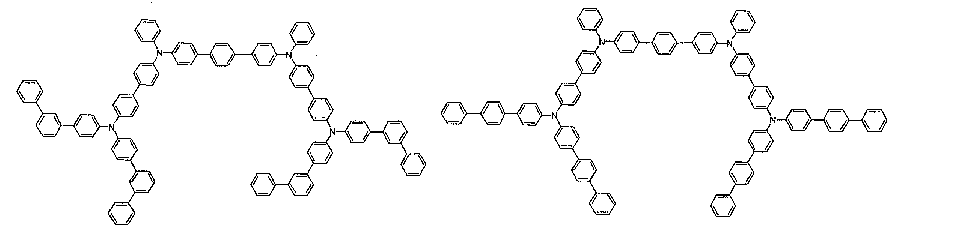

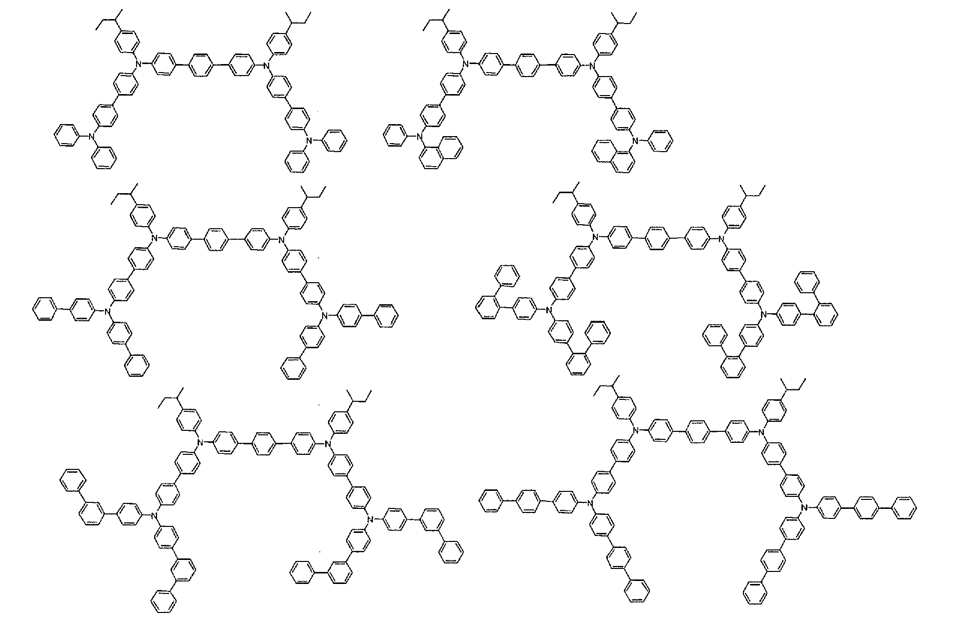

It is preferable that the aromatic amine compound of the present invention is a material for the organic EL device, particularly is appropriate as a hole transporting material or a hole injecting material both for the organic EL device. At least one layer among the organic thin film layers in the organic EL device of the present invention contains the aromatic amine compound. It is preferable that the above aromatic amine compound is contained particularly in a hole transporting region or a hole injecting region, further that the compound is a main component. The aromatic amine compound is made to be contained into the hole transporting region or the hole injecting region, and more preferably, a light emitting layer should be formed in accordance with a wet process, thereby fabricating the organic EL device. Further, the present invention provides a thin- film-forming solution for the organic EL device containing the aromatic amine compound in an amount of 0.1 % by mass or more.

Following is a description regarding a device structure about the organic EL device of the present invention.

### (I) Construction of the organic EL device

Typical examples of the construction in the organic EL device of the present invention are shown below.
(1) Anode / light emitting layer / cathode
(2) Anode / hole injecting layer / light emitting layer / cathode
(3) Anode / light emitting layer / electron injecting layer / cathode
(4) Anode / hole injecting layer / light emitting layer / electron injecting layer / cathode
(5) Anode / organic semiconductor layer / light emitting layer / cathode
(6) Anode / organic semiconductor layer / electron barrier layer / light emitting layer / cathode
(7) Anode / organic semiconductor layer / light emitting layer / adhesion improving layer / cathode
(8) Anode / hole injecting layer / hole transporting layer / light emitting layer / electron injecting layer / cathode
(9) Anode / insulating layer / light emitting layer / insulating layer / cathode
(10) Anode / inorganic semiconductor layer / insulating layer / light emitting layer / insulating layer / cathode
(11) Anode / organic semiconductor layer / insulating layer / light emitting layer / insulating layer / cathode
(12) Anode / insulating layer / hole injecting layer / hole transporting layer / light emitting layer / insulating layer / cathode
(13) Anode / insulating layer / hole injecting layer / hole transporting layer / light emitting layer / electron injecting layer / cathode
   Among the above constructions, construction (8) is usually preferable though not limited to.
   Although the aromatic amine derivative of the present invention may be used to any organic thin film layer in the organic EL device, it is employable in the light emitting region or the hole transporting region, and a preferable embodiment of employing it into the hole transporting region, or a particularly preferable embodiment of employing it into a hole transporting layer will make crystallization of molecules hard to cause thereby enhancing yield of producing the organic EL device.
   In the organic EL device of the present invention, the organic thin film layer preferably contains the aromatic amine derivative of the present invention in an amount of 30 to 100 % by mole.

### (II) Light-transmitting substrate

In general, the organic EL device is fabricated on a light-transmitting substrate. The light-transmitting substrate is a substrate for supporting the organic EL device and preferably a flat and smooth substrate having a light transmittance of 50 % or greater to visible light of 400 to 700 nm.
As the light-transmitting substrate, for example, glass plate and synthetic resin plate are advantageously employed. Specific examples of the glass plate include soda lime glass, glass containing barium and strontium, lead glass, aluminosilicate glass, borosilicate glass, barium borosilicate glass and quartz. Specific examples of the synthetic resin plate include plate made of polycarbonate resins, acrylic resins, polyethylene telephthalate resins, polyether sulfide resins and polysulfone resins.

### (III) Anode

The anode in the organic EL device of the present invention has a function of injecting holes into a hole transporting layer or a light emitting layer, and it is effective that the anode has a work function of 4.5 eV or greater. Specific examples of the material for the anode include indium tin oxide alloy (ITO), tin oxide (NESA), indium-zinc oxide alloy (IZO), gold, silver, platinum, copper, etc.
The anode can be prepared by forming a thin film of the electrode material described above in accordance with a process such as the vapor deposition process and the sputtering process.
When the light emitted from the light emitting layer is obtained through the anode, it is preferable that the anode has a transmittance of the emitted light greater than 10 %. It is also preferable that the sheet resistivity of the anode is several hundreds Ω/□ or smaller. The thickness of the anode is, in general, selected usually in the range of from 10 nm to 1 µm and preferably in the range of from 10 to 200 nm.

### (IV) Light emitting layer

In the organic EL device of the present invention, the light emitting layer combines the following functions (1) to (3):
(1) The injecting function: the function of injecting holes from the anode or the hole injecting layer and injecting electrons from the cathode or the electron injecting layer when an electric field is applied;
(2) The transporting function: the function of transporting the injected charges (electrons and holes) by the force of the electric field; and
(3) The light emitting function: the function of providing the field for recombination of electrons and holes and promote the recombination to emit light.
   Although there may be a difference between the capability of the holes being injected and the capability of the electrons being injected, and although there may be a difference between the transporting functions expressed by mobilities of the holes and the electrons, either one of the charges is preferable to be transferred.
   As the process for forming the light emitting layer, a well-known process such as the vapor deposition process, the spin coating process and the LB process can be employed. It is particularly preferable for the light emitting layer to be a molecular deposit film. The molecular deposit film is a thin film formed by the deposition of a material compound in the gas phase or a thin film formed by the solidification of a material compound in a solution or liquid phase. In general, the molecular deposit film can be distinguished from the thin film formed in accordance with the LB process (the molecular accumulation film) based on the differences in the aggregation structure and higher order structures and functional differences caused by these structural differences.
   In addition, as disclosed in JP 57-51781A, the light emitting layer can also be formed by dissolving a binder such as a resin and the material compounds into a solvent to prepare a solution, followed by forming a thin film from the prepared solution in accordance with the spin coating process or the like.
   In the present invention, any well-known light emitting material other than the aromatic amine derivative of the present invention may be contained in the light emitting layer, or a light emitting layer containing any other well-known light emitting material may be laminated with the light emitting layer containing the aromatic amine derivative of the present invention, as long as the object of the present invention is not adversely affected.

Light emitting materials or doping materials to be used for the light emitting layer together with the aromatic amine derivatives of the present invention includes, for example, anthracene, naphthalene, phenanthrene, pyrene, tetracene, coronene, chrysene, fluorescein, perylene, phthaloperylene, naphthaloperylene, perinone, phthaloperinone, naphthaloperinone, diphenylbutadiene, tetraphenylbutadiene, coumarin, oxadiazole, aldazine, bisbenzooxazoline, bisstyryl, pyrazine, cyclopentadiene, quinoline metal complex, aminoquinoline metal complex, benzoquinoline metal complex, imine, diphenylethylene, vinylanthracene, diaminecarbazol, pyran, thiopyran, polymethyne, merocyanine, imidazol chelate oxinoid compound, quinacridone, rubrene and fluorescent dye, but not limited thereto.

Preferable host materials to be used for the light emitting layer together with the aromatic amine derivatives of the present invention include compounds represented by following general formulae (i) to (ix).
An asymmetric anthracene represented by the following general formula (i): 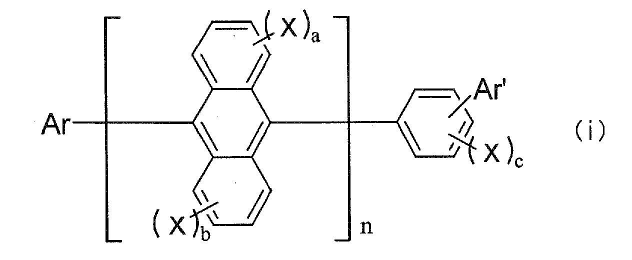 (In the above formula, Ar represents a substituted or unsubstituted fused aromatic group having 10 to 50 ring carbon atoms; Ar' represents a substituted or unsubstituted aromatic group having 6 to 50 ring carbon atoms; X represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group or a hydroxyl group; a, b and c each represents an integer of 0 to 4; n represents an integer of 1 to 3; with the proviso that when n is an integer of 2 or greater, the plural groups within square brackets ( [ ] ) may be the same with or different from each other.)

An asymmetric monoanthracene derivative represented by the following general formula (ii): 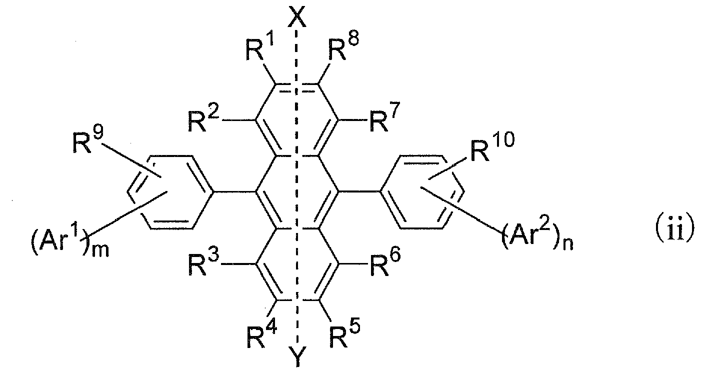 (In the formula, Ar¹ and Ar² each independently represents a substituted or unsubstituted aromatic ring group having 6 to 50 ring carbon atoms; m and n each represents an integer of 1 to 4; with the proviso that in a case where m=n=1 and each bonding position of Ar¹ and Ar² to a benzene ring is bilaterally symmetric to each other, Ar¹ is different from Ar², or that there is not a case where a group symmetric to X-Y axis bonds to 1- to 8-positions of anthracene ring; and in a case where m or n represents an integer of 2 to 4, m is different from n; R¹ to R¹⁰ each independently represents a hydrogen atom, a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group and a hydroxyl group.)

An asymmetric pyrene derivative represented by the following general formula (iii): 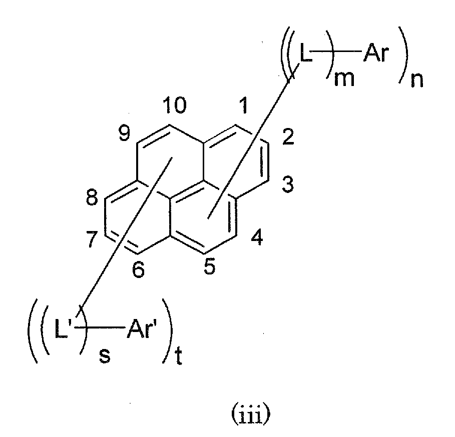 [In the general formula, Ar and Ar' each represents a substituted or unsubstituted aromatic group having 6 to 50 ring carbon atoms; L and L' each represents a substituted or unsubstituted phenylene group, a substituted or unsubstituted naphthalenylene group, a substituted or unsubstituted fluorenylene group or a substituted or unsubstituted dibenzosilolylene group; m represents an integer of 0 to 2, n represents an integer of 1 to 4, s represents an integer of 0 to 2 and t represents an integer of 0 to 4; L or Ar is bonded to any one of 1- to 5-positions of pyrene ring; and L' or Ar' is bonded to any one of 6- to 10-positions of pyrene ring; with the proviso that when n+t represents an even number, Ar, Ar', L and L' satisfy the following conditions (1) or (2):
(1) Ar ≠ Ar' and/or L ≠ L' (wherein ≠ means that each group has a different structure)
(2) when Ar = Ar' and L = L'
(2-1) m ≠ s, and/or n ≠ t, or
(2-2) when m = s and n = t,
(2-2-1) L and L', or the pyrene ring, are respectively bonded to different positions of Ar and Ar¹, or
(2-2-2) in the case where L and L', or the pyrene ring, are respectively bonded to the same position of Ar and Ar', the case where L and L', or Ar and Ar' are bonded to the 1- and 6-positions or 2- and 7-positions of the pyrene ring is excluded.]

An asymmetric anthracene derivative represented by the following general formula (iv): 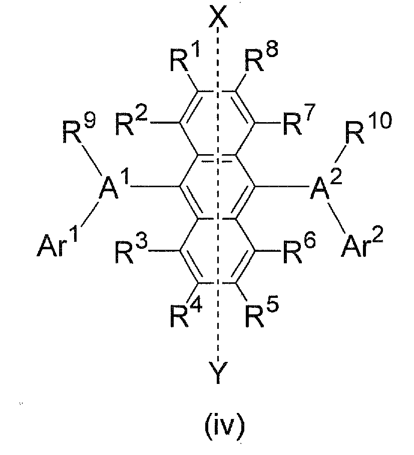 (In the formula, A¹ and A² each independently represents a substituted or unsubstituted fused aromatic ring group having 10 to 20 ring carbon atoms; Ar¹ and Ar² each independently represents a hydrogen atom, or a substituted or unsubstituted aromatic ring group having 6 to 50 ring carbon atoms.

R¹ to R¹⁰ each independently represents a hydrogen atom, a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group and a hydroxyl group.

The number of each of Ar¹, Ar², R⁹ and R¹⁰ may be two or more, and two neighboring groups thereof may form a saturated or unsaturated ring structure; with the proviso that the groups at 9- and 10-positions of the central anthracene are not symmetrical with respect to the X-Y axis.)

An anthracene derivative represented by the following general formula (v): 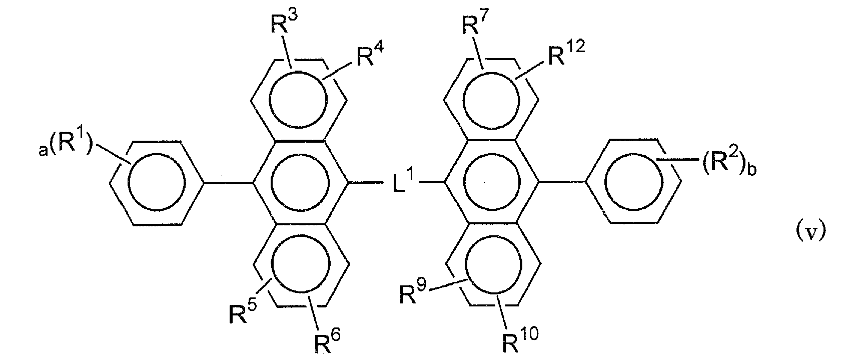 (In the formula, R¹ to R¹⁰ each independently represents a hydrogen atom, an alkyl group, a cycloalkyl group, an aryl group which may be substituted, an alkoxyl group, an aryloxy group, an alkylamino group, an alkenyl group, an arylamino group or a heterocyclic group which may be substituted; a and b each represents an integer of 1 to 5, and when any of a and b is 2 or greater, plural R¹ or R² may be the same with or different from each other, additionally plural R¹ or R² may bond each other to form a ring; any pair of R³ and R⁴, R⁵ and R⁶, R⁷ and R⁸, and R⁹ and R¹⁰ may bond each other to form a ring; L¹ represents a single bond, -O-, -S-, -N(R), an alkylene group or an arylene group, wherein R represents an alkyl group, or an aryl group which may be substituted.)

An anthracene derivative represented by the following general formula (vi) : 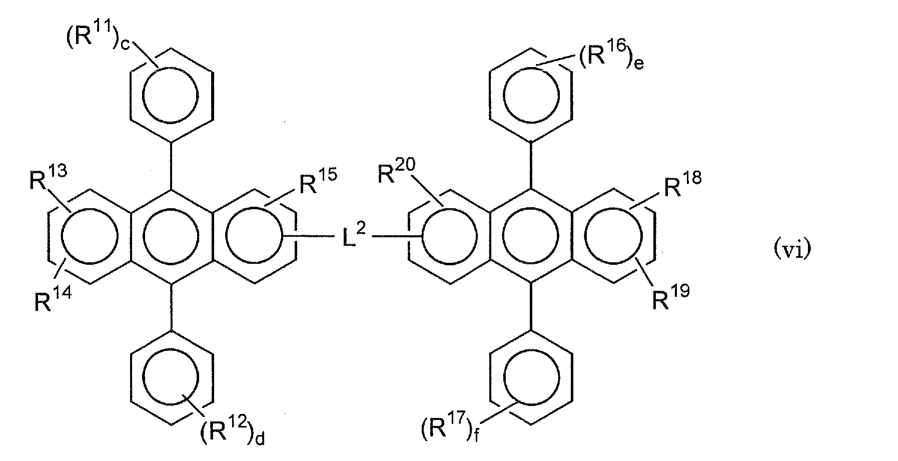 (In the formula, R¹¹ to R²⁰ each independently represents a hydrogen atom, an alkyl group, a cycloalkyl group, an aryl group, an alkoxyl group, an aryloxy group, an alkylamino group, an arylamino group or a heterocyclic group which may be substituted; c, d, e and f each represents an integer of 1 to 5, and when they are 2 or greater, plural R¹¹, R¹², R¹⁶ or R¹⁷ may be the same with or different from each other, additionally plural R¹¹, R¹², R¹⁶ or R¹⁷ may bond each other to form a ring; any pair of R¹³ with R¹⁴ and R¹⁸ with R¹⁹ may bond each other to form a ring, L² represents a single bond, -O-, -S-, -N(R), an alkylene group or an arylene group; wherein R represents an alkyl group, or an aryl group which may be substituted.)

A spirofluorene derivative represented by the following general formula (vii): 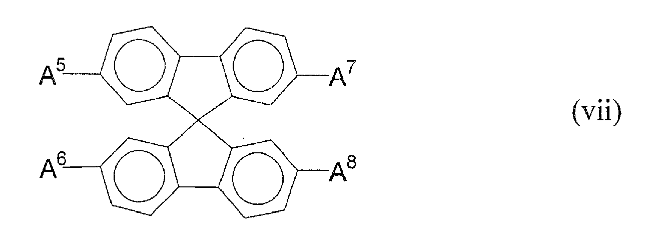 (In the formula, A⁵ to A⁸ each independently represents a substituted or unsubstituted biphenylyl group or a substituted or unsubstituted naphthyl group.)

A compound containing a fused ring represented by the following general formula (viii): 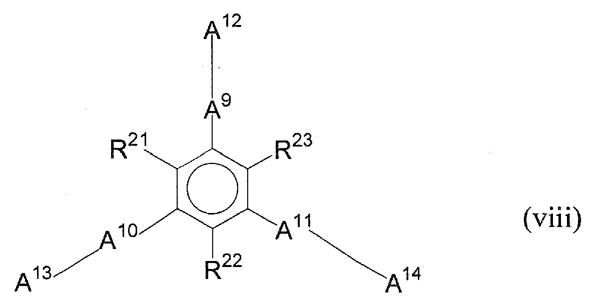 (In the formula, A⁹ to A¹⁴ are the same as described above, and R²¹ to R²³ each independently represents a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 carbon atoms, an aralkyloxy group having 7 to 18 carbon atoms, an arylamino group having 5 to 16 carbon atoms, a nitro group, a cyano group, an ester group having 1 to 6 carbon atoms or a halogen atom; and at least one of A⁹ to A¹⁴ represents a fused aromatic ring having 3 or more rings.)

A fluorene compound represented by the following general formula (ix): 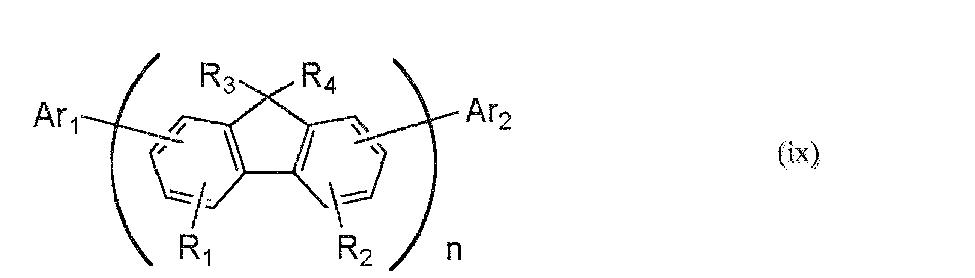 (In the formula, R₁ and R₂ each independently represents a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group or a halogen atom; R₁'s and R₂'s bonding to different fluorene groups may be respectively the same or different, and R₁ and R₂ bonding to the same fluorene group may be the same or different; R₃ and R₄ each independently represents a hydrogen atom, a substituted or unsubstituted alkyl group, a substituted or unsubstituted aralkyl group, a substituted or unsubstituted aryl group, or a substituted or unsubstituted heterocyclic group, and R₃'s and R₄'s bonding to different fluorene groups may be respectively the same or different, and R₃ and R₄ bonding to the same fluorene group may be the same or different; Ar₁ and Ar₂ each independently represents a substituted or unsubstituted fused polycyclic aromatic group consisting of benzene rings of 3 or more or a substituted or unsubstituted fused polycyclic heterocyclic group comprising of benzene rings and hetero rings of 3 or more in total; and further, Ar₁ and Ar₂ may be the same with, or different from each other; and n represents an integer of 1 to 10.)

Among the above host materials, an anthracene derivative is preferable and a monoanthracene derivative is more preferable, further an asymmetric anthracene is particularly preferable.
In addition, a phosphorescent compound may be employed as a light emitting material for dopant. A compound containing a carbazole ring for a host material is preferable as the phosphorescent compound. The dopant is not limited as long as it is a compound capable of emitting light from triplet exciton, and preferably a metal complex containing at least one metal selected from the group consisting of Ir, Ru, Pd, Pt, Os and Re, more preferably a porphyrin metal complex or an ortho-metallated complex.
A suitable host for phosphorescence composed of a compound containing a carbazole ring is a compound having a function of making the phosphorescent compound to emit light by the energy transfer from its excitation state to the phosphorescent compound. The host compound is not limited as long as capable of transferring the exciton energy to the phosphorescent compound and may be appropriately selected according to the purpose. The host compound may have any group such as a hetero ring in addition to the carbazole ring.

Specific examples of the host compound include a carbazole derivative, a triazole derivative, an oxazole derivative, an oxadiazole derivative, an imidazole derivative, a polyarylalkane derivative, a pyrazoline derivative, a pyrazlone derivative, a phenylenediamine derivative, an arylamine derivative, a chalcone derivative substituted by amino group, a styrylanthracene derivative, a fluorenone derivative, a hydrazone derivative, a stilbene derivative, a silazane derivative, an aromatic tertiary amine compound, a styrylamine compound, an aromatic dimethylidene compound, a porphyrin-based compound, an anthraquinodimethane derivative, an anthrone derivative, a diphenylquinone derivative, a thiopyrandioxide derivative, a carbodimide derivative, a fluorenylidene methane derivative, a distyrylpyrazine derivative, heterocyclic tetracarboxylic anhydride such as a naphthaleneperylene, a phthalocyanine derivative, a metal complex of 8-quinolinol derivative, various metal complex polysilane compound such as a metal complex having a ligand of metallophthalocyanine, benzoxazole or benzothiazole, an electrically conductive polymeric oligomer such as a poly(N-vinylcarbazole) derivative, an aniline copolymer, a thiophene oligomer and a polythiophene, polymer compound such as a polythiophene derivative, a polyphenylene derivative, a polyphenylenevinylene derivative and a polyfluorene derivative. The host compound may be used alone or in combination of two or more.
More specific examples include the following:

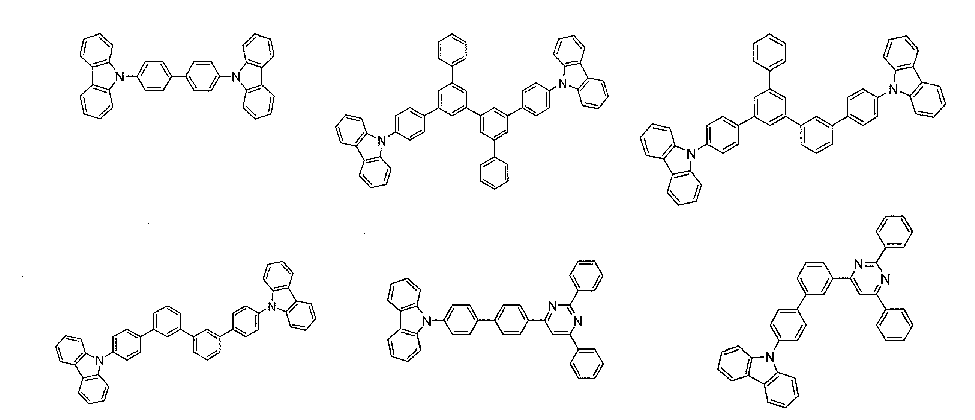

The phosphorescent dopant is a compound capable of emitting light from the triplet exciton. The phosphorescent dopant is not restricted as long as it emits light from the triplet exciton, and preferably a metal complex containing at least one metal selected from the group consisting of Ir, Ru, Pd, Pt, Os and Re, more preferably a porphyrin metal complex or an ortho-metallated metal complex. As the porphyrin metal complex, a porphyrin platinum complex is preferable. The phosphorescent compound may be used alone or in combination of two more.
There are various ligands to form the ortho-metallated metal complex, and preferred are 2-phenylpyridine derivatives, 7,8-benzoquinoline derivatives, 2-(2-thienyl)pyridine derivatives, 2-(1-naphthyl)pyridine derivatives, and 2-phenylquinoline derivatives, etc. The derivatives may have a substituent as occasion demands. In particular, a dopant introduced with a fluorine atom or a trifluoromethyl group is preferable for the blue light emission. In addition, a ligand other than the above ligands such as acetylacetonate and picrate may be introduced as an auxiliary ligand.
The amount of the phosphorescent dopant in the light emitting layer may be selected for the objective as appropriate without particularly restricted, and for example, it may be selected in the range of from 0.1 to 70 % by mass, preferably in the range of from 1 to 30 % by mass. The emission is faint and the advantage is not demonstrated when the amount is less than 0.1 % by mass. The concentration quenching becomes noticeable so that the device performance is deteriorated when the amount exceeds 70 % by mass.
Further, the light emitting layer may contain a hole transporting material, a electron transporting material or a polymer binder, if necessary.
The thickness of the light emitting layer is, in general, selected in the range of from 5 to 50 nm, preferably in the range of from 7 to 50 nm and the most preferably in the range of from 10 to 50 nm. It is resulted in difficult to form the light emitting layer and to control chromaticity thereof when the thickness is thinner than 5 nm, and it may be resulted in possibility of elevating driving voltage when it exceeds 50 nm.

### (V) Hole injecting and transporting layer (hole transporting region)

The hole injecting and transporting layer is a layer which helps the injection of holes into the light emitting layer and transports the holes to the light emitting region. The layer exhibits a great mobility of holes and, in general, has an ionization energy as small as 5.5 eV or smaller. For the hole injecting and transporting layer, a material which transports holes to the light emitting layer at a small strength of the electric field is preferable. A material which exhibits, for example, a mobility of holes of at least 10⁻⁴ cm²/V**.**sec under an electric field of from 10⁴ to 10⁶ V/cm is preferable.
When the aromatic amine derivative of the present invention is employed in the hole transporting region, the hole injecting and transporting layer may be composed of the aromatic amine derivative of the present invention alone or in combination with another material.
With regard to the material which may be employed for forming the hole injecting and transporting layer in combination with the aromatic amine derivative of the present invention, any material having the foregoing preferable properties is employed without particularly restricted, which is selected from compounds commonly used as a hole transporting material of photoconductive materials and compounds used for forming the hole injecting and transporting layer of EL devices.

Specific examples include triazole derivatives (refer to U.S. Pat. No. 3,112,197, etc.), oxadiazole derivatives (refer to U.S. Pat. No. 3,189,447, etc.), imidazole derivatives (refer to JP-B 37-16096, etc.), polyarylalkane derivatives (refer to U.S. Pat. Nos. 3,615,402; 3,820,989; 3,542,544, JP-B 45-555, JP-B 51-10983, JP 51-93224A, JP 55-17105A, JP 56-4148A, JP 55-108667A, JP 55-156953A, JP 56-36656A, etc.), pyrazoline derivatives and pyrazolone derivatives (refer to U.S. Pat. Nos. 3,180,729; 4,278,746; JP 55-88064A, JP 55-88065A, JP 49-105537A, JP 55-51086A, JP 56-80051A, JP 56-88141A, JP 57-45545A, JP 54-112637A, JP 55-74546A, etc.), phenylenediamine derivatives (Refer to U.S. Pat. No. 3,615,404; JP-B 51-10105, JP-B 46-3712, JP-B 47-25336, JP 54-53435A, JP 54-110536A, JP 54-119925A, etc.), arylamine derivatives (refer to U.S. Pat. Nos. 3,567,450; 3,180,703; 3,240,597; 3,658,520; 4,232,103; 4,175,961; 4,012,376; JP-B 49-35702, JP-B 39-27577, JP 55-144250A, JP 56-119132A, JP 56-22437A, German Patent No. 1,110,518, etc.), amino-substituted chalcone derivatives (refer to U.S. Pat. No. 3,526,501, etc.), oxazole derivatives (disclosed in U.S. Pat. No. 3,257,203, etc.), styrylanthracene derivatives (refer to JP 56-46234A, etc.), fluorenone derivatives (refer to JP 54-110837A, etc.), hydrazone derivatives (refer to U.S. Pat. No. 3,717,462, JP 54-59143A, JP 55-52063A, JP 55-52064A, JP 55-46760A, JP 55-85495 A, JP 57-11350A, JP 57-148749A, JP 2-311591A, etc.), stilbene derivatives (refer to JP 61-210363A, JP 61-228451A, JP 61-14642A, JP 61-72255A, JP 62-47646A, JP 62-36674A, JP 62-10652A, JP 62-30255A, JP 60-93455A, JP 60-94462A, JP 60-174749A, JP 60-175052A, etc.), silazane derivatives (U.S. Pat. No. 4,950,950), polysilane-based polymer (JP 2-204996A), aniline-based copolymer (JP 2-282263A), an electrically conductive polymer oligomer disclosed in JP 1-211399A (particularly, thiophene oligomer), etc.

With regard to the material for the hole injecting and transporting layer, the above materials are also employable, and porphyrin compounds (disclosed in JP 63-2956965A), aromatic tertiary amine compounds and styryl amine compounds (refer to U.S Pat. No. 4,127,412, JP 53-27033A, JP 54-58445A, JP 54-149634A, JP 54-64299A, JP 55-79450A, JP 55-144250A, JP 56-119132A, JP 61-295558A, JP 61-98353A, JP 63-295695A, etc.) are preferable and the aromatic tertiary amine compounds are particularly preferable.
Further examples include, 4,4'-bis(N-(1-naphthyl)-N-phenylamino)biphenyl (NPD) which has 2 fused aromatic rings in its molecule described in U.S Pat. No. 5,061,569 and 4,4',4"-tris(N-(3-methylphenyl)-N-phenylamino)triphenylamine (MTDATA) described in JP 4-308688A which includes three triphenylamine units connected in a star burst configuration.
In addition to the above-mentioned aromatic dimethylidene compound described as a material for the light emitting layer, inorganic compound such as p-type Si and p-type SiC may be used as the material for the hole injecting and transporting layer.

To form the hole injecting and transporting layer, a thin film can be formed from the aromatic amine derivative of the present invention in accordance with a well-known process such as the vacuum vapor deposition process, the spin coating process, the casting process and the LB process. Although the thickness of the hole injecting and transporting layer is not particularly limited, the thickness is usually from 5 nm to 5 µm. The hole injecting and transporting layer may be a single layer made of one or more kinds of materials mentioned above or may be laminated with another hole injecting and transporting layer made of a different compound, as long as the hole injecting and transporting layer contains the aromatic amine derivative of the present invention in its hole transporting region. An organic semiconductor layer which preferably has an electric conductance of 10⁻¹⁰ S/cm or greater may be provided to assist the injection of holes or electrons into the light emitting layer. Examples of the materials for the organic semiconductor layer include electrically conductive oligomers such as an oligomer having thiophene and an oligomer having arylamine disclosed in JP 8-193191A; and electrically conductive dendrimers such as a dendrimer having an arylamine dendrimer.

### (VI) Electron injecting and transporting layer

The electron injecting and transporting layer assists the injection of electrons into the light emitting layer and transports them to a light emitting region, and it is a layer having a great electron mobility. Among the electron injecting layers, the adhesion improving layer is a layer made of a material exhibiting excellent adhesion to the cathode.
Further, it is known that because the emitted light reflects on the electrode (cathode in this case) in the organic EL device, the light taken out directly through the anode and the light taken out after the reflection on the electrode interferes each other. To utilize the interference effect effectively, the thickness of the electron transporting layer is appropriately selected from several nm to several µm. When the film is thicker, the hole mobility is preferably at least 10⁻⁵ cm²/V·s under an electric field from 10⁴ to 10⁶ V/cm for avoiding the elevation of driving voltage.
As the material for the electron injecting layer, metal complexes of 8-hydroxyquinoline or derivatives thereof and oxadiazole derivatives are preferable. Examples of the metal complexes of 8-hydroxyquinoline and derivatives thereof include metal chelate oxinoid compounds including chelates of oxine (in general, 8-quinolinol or 8-hydroxyquinoline), for example, tris(8-quinolinolato)aluminum.

Examples of the oxadiazole derivatives include an electron transfer compound represented by the following general formulae: 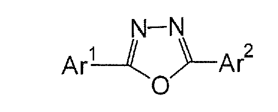 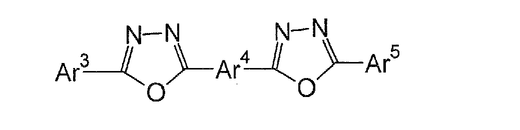 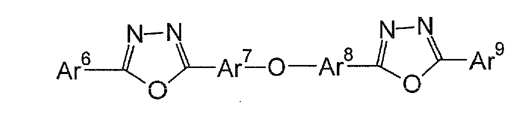 (In the formula, Ar¹, Ar², Ar³, Ar⁵, Ar⁶ and Ar⁹ each independently represents a substituted or unsubstituted aryl group which may be the same or different; Ar⁴, Ar⁷ and Ar⁸ each independently represents a substituted or unsubstituted arylene group which may be the same or different.)
Examples of aryl group include a phenyl group, a biphenyl group, an anthranyl group, a perilenyl group and a pyrenyl group. Examples of the arylene group include a phenylene group, a naphthylene group, a biphenylene group, an anthranylene group, a perilenylene group, a pyrenylene group, etc. Examples of the substituent include an alkyl group having 1 to 10 carbon atoms, an alkoxy group having 1 to 10 carbon atoms and a cyano group. The electron transfer compound is preferably a thin-film forming compound.

Specific examples of the electron transfer compounds are shown below: 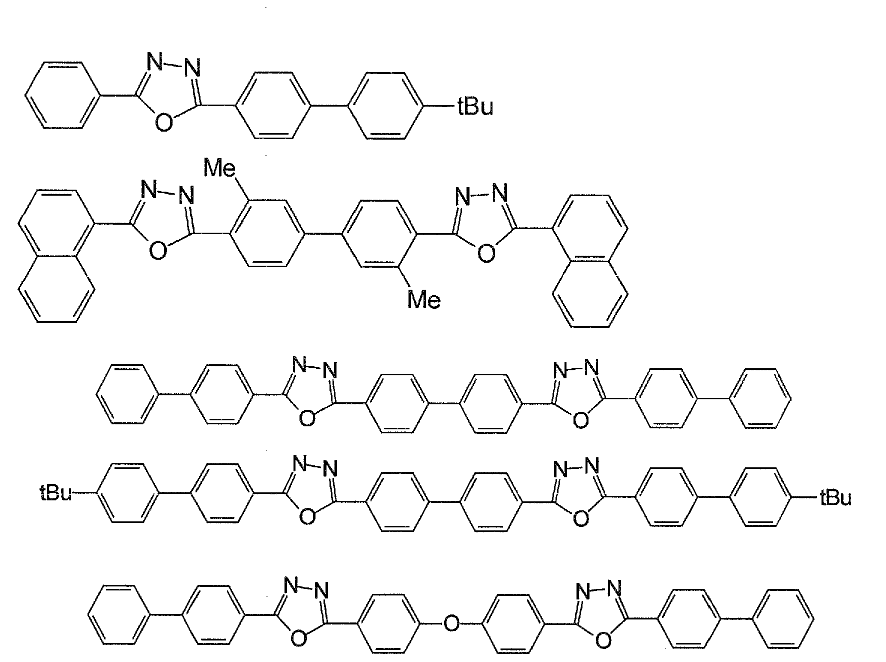 Further, materials shown by following general formulae (A) to (E) are employable for the electron injecting layer and the electron transporting layer.

A nitrogen-containing heterocyclic derivative represented by the following general formula (A) or (B): 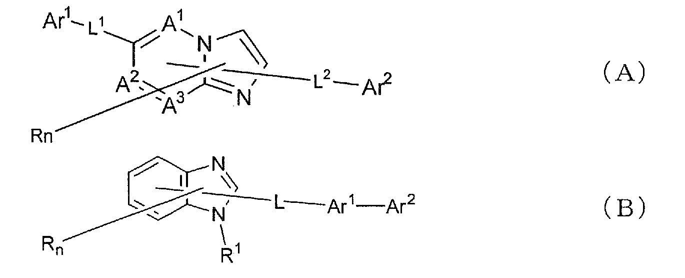 (In the general formulae (A) and (B), A¹ to A³ each independently represents a nitrogen atom or a carbon atom; Ar¹ represents a substituted or unsubstituted aryl group having 6 to 60 ring carbon atoms or a substituted or unsubstituted heteroaryl group having 3 to 60 ring carbon atoms; Ar² represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, a substituted or unsubstituted alkoxy group having 1 to 20 carbon atoms or those divalent groups. At least one of Ar¹ and Ar² represents a substituted or unsubstituted fused ring group having 10 to 60 ring carbon atoms, or a substituted or unsubstituted monohetero fused ring group having 3 to 60 ring carbon atoms.
L¹, L² and L each independently represents a single bond, a substituted or unsubstituted arylene group having 6 to 60 ring carbon atoms, a substituted or unsubstituted heteroarylene group having 3 to 60 ring carbon atoms or a substituted or unsubstituted fluorenylene group.
R represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms; n represents an integer of 0 to 5; when n is 2 or greater, plural R's may be the same or different and adjacent couple of R's may bond to form a carbocyclic aliphatic ring or a carbocyclic aromatic ring.)

A nitrogen-containing heterocyclic derivative represented by a following general formula (C):

HAr-L-Ar¹-Ar² (C)

(In the formula, HAr represents a nitrogen-containing heterocyclic group having 3 to 40 carbon atoms which may have a substituent; L represents a single bond, an arylene group having 6 to 60 carbon atoms which may have a substituent, a heteroarylene group having 3 to 60 carbon atoms which may have a substituent; Ar¹ represents a divalent aromatic hydrocarbon group having 6 to 60 carbon atoms which may have a substituent; and Ar² represents an aryl group having 6 to 60 carbon atoms which may have a substituent or a heteroaryl group having 3 to 60 carbon atoms which may have a substituent.)

A silacyclopentadiene derivative represented by a following general formula (D):

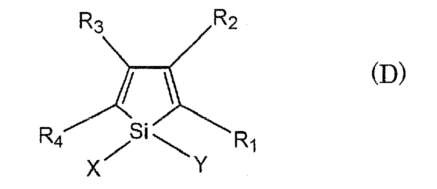

(In the formula, X and Y each independently represents a saturated or unsaturated hydorocarbon group having 1 to 6 carbon atoms, an alkoxy group, an alkenyloxy group, an alkynyloxy group, a hydroxy group, a substituted or unsubstituted aryl group, or a substituted or unsubstituted hetero ring, or a structure in which X and Y bond to each other to form a saturated or unsaturated ring; R₁ to R₄ each independently represents a hydrogen atom, a halogen atom, a substituted or unsubstituted alkyl group having 1 to 6 carbon atoms, an alkoxy group, an aryloxy group, a perfluoroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, a carbamoyl group, an aryl group, a hetero ring group, an alkenyl group, an alkynyl group, a nitro group, a formyl group, a nitroso group, a formyloxy group, an isocyano group, a cyanate group, an isocyanate group, a thiocyanate group, an isothiocyanate group, or a cyano group, or a structure in which an adjacent pair of R₁ to R₄ bond each other to form a substituted or unsubstituted fused ring.)

A borane derivative represented by the following general formula (E):

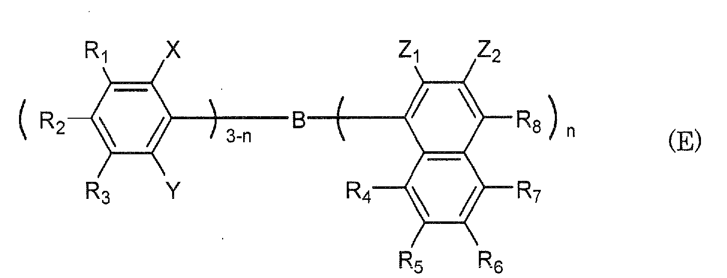

(In the formula, R₁ to R₈ and Z₂ each independently represents a hydrogen atom, a saturated or unsaturated hydrocarbon group, an aromatic group, a hetero ring group, substituted amino group, a substituted boryl group, an alkoxy group or an aryloxy group; X, Y and Z₁ each independently represents a saturated or unsaturated hydrocarbon group, an aromatic group, a hetero ring group, substituted amino group, an alkoxy group or an aryloxy group; substituents of Z₁ and Z₂ may bonds to each other to form a fused ring; n represents an integer of 1 to 3; and when n is 2 or greater, Z₁'s may be different from each other; with the proviso that a case where n is 1, X, Y and R₂ are methyl groups and R₈ is a hydrogen atom or a substituted boryl group and a case where n is 3 and Z₁ is a methyl group are excluded.)

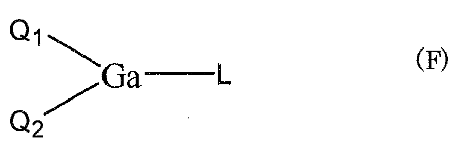

[In the formula, Q¹ and Q² each independently represents a ligand represented by the following general formula (G), L represents a halogen atom, a substituted or unsubstituted alkyl group, a substituted or unsubstituted cycloalkyl group, a substituted or unsubstituted aryl group, a substituted or unsubstituted heterocyclic group, -OR¹ wherein R¹ represents a hydrogen atom, a substituted or unsubstituted alkyl group, a substituted or unsubstituted cycloalkyl group, a substituted or unsubstituted aryl group or a substituted or unsubstituted heterocyclic group; or a ligand represented by -O-Ga-Q³(Q⁴) wherein Q³ and Q⁴ are the same as Q¹ and Q².]

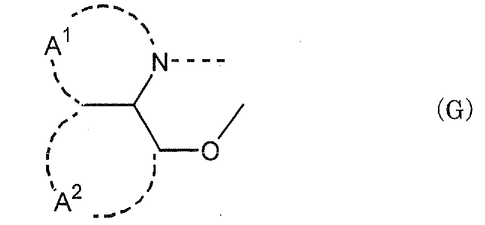

[In the formula, rings A¹ and A² represent fused six-membered aryl ring structures which may be substituted.]

The metal complex strongly characterizes n-type semiconductor and has a large capability of the electron injection. Since the fromation energy for forming the metal complex is small, the bonding between the metal and the ligand is strong, to increase the fluorescence quantum efficiency of light emitting materials
Specific examples of the substituents of rings A¹ and A² each forming the ligand in the general formula (G) include halogen atom such as chlorine atom, bromine atom, iodine atom and fluorine atom; substituted or unsubstituted alkyl group such as methyl group, ethyl group, propyl group, butyl group, s-butyl group, t-butyl group, pentyl group, hexyl group, heptyl group, octyl group, stearyl group, trichloromethyl group, etc.; substituted or unsubstituted aryl group such as phenyl group, naphthyl group, 3-methylphenyl group, 3-methoxyphenyl group, 3-fluorophenyl group, 3-trichloromethylphenyl group, 3-trifluoromethylphenyl group, 3-nitrophenyl group, etc.; substituted or unsubstituted alkoxy group such as methoxy group, n-butoxy group, t-butoxy group, trichloromethoxy group, trifluoroethoxy group, pentafluoropropoxy group, 2,2,3,3-tetrafluoropropoxy group, 1,1,1,3,3,3-hexafluoro-2-propoxy group, 6-(perfluoroethyl)hexyloxy group, etc.; substituted or unsubstituted aryloxy group such as phenoxy group, p-nitrophenoxy group, p-t-butylphenoxy group, 3-fluorophenoxy group, pentafluorophenyl group, 3-trifluoromethylphenoxy group, etc.; substituted or unsubstituted alkylthio group such as methylthio group, ethylthio group, t-butylthio group, hexylthio group, octylthio group, trifluoromethylthio group, etc.; substituted or unsubstituted arylthio group such as phenylthio group, p-nitrophenylthio group, p-t-butylphenylthio group, 3-fluorophenylthio group, pentafluorophenylthio group, 3-trifluoromethylphenylthio group, etc.; cyano group; nitro group; amino group; mono- or di-substituted amino groups such as methylamino group, diethylamino group, ethylamino group, diethylamino group, dipropylamino group, dibutyl amino group, diphenylamino group, etc.; acylamino groups such as bis(acetoxymethyl)amino group, bis(acetoxyethyl)amino group, bis(acetoxypropyl)amino group, bis(acetoxybutyl) amino group, etc.; hydroxy group; siloxy group; acyl group; carbamoyl group such as methylcarbamoyl group, dimethylcarbamoyl group, ethylcarbamoyl group, diethylcarbamoyl group, a propylcarbamoyl goup, butylcarbamoyl group, a phenylcarbamoyl group, etc.; carboxylic acid group; sulfonic acid group; imido group, cycloalkyl group such as cyclopentyl group, cyclohexyl group, etc.; aryl group such as phenyl group, naphthyl group, biphenyl group, anthryl group, phenanthryl group, fluorenyl group, pyrenyl group, etc.; heterocyclic group such as pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, triazinyl group, carbazolyl group, furanyl group, thiophenyl group, oxazolyl group, an oxadiazolyl group, a benzoxazolyl group, a thiazolyl group, a thiadiazolyl group, benzothiazolyl group, triazolyl group, imidazolyl group, benzimidazolyl group, pranyl group, etc. The above substituents may bond each other to form a six-membered aryl ring or hetero ring.

A preferred embodiment of the organic EL device of the present invention contains a reductive dopant in an electron transporting region or an interfacial region between a cathode and an organic compound layer. The reductive dopant is defined as the substance capable of reducing an electron transporting compound. Accordingly, various compounds having a specified reducing property may be employable and examples of the reductive dopant include at least one compound selected from alkali metals, alkaline earth metals, rare earth metals, oxides of alkali metals, halides of alkali metals, oxides of alkaline earth metals, halides of alkaline earth metals, oxides of rare earth metals, halides of rare earth metals, organic complexes of alkali metals, organic complexes of alkaline earth metals, and organic complexes of rare earth metals.
Examples of the preferable reductive dopant include at least one alkali metal selected from a group consisting of Na (the work function: 2.36 eV), K (the work function: 2.28 eV), Rb (the work function: 2.16 eV) and Cs (the work function: 1.95 eV) or at least one alkaline earth metals selected from a group consisting of Ca (the work function: 2.9 eV), Sr (the work function: 2.0 to 2.5 eV) and Ba (the work function: 2.52 eV). A reductive dopant having a work function of 2.9 eV or smaller is particularly preferable. Among those, more preferable reductive dopants include at least one kind or more alkali metal selected from the group consisting of K, Rb and Cs, the latter Rb or Cs being further more preferable and the last Cs being the most preferable. Since those alkali metals have a particularly high reducing capability, the luminance is improved and the lifetime is prolonged by the addition thereof into an electron injection region in a relatively small amount. A combination of two or more alkali metals is also preferably used as the reductive dopant having a work function of 2.9 eV or smaller. A combination containing Cs such as Cs and Na, Cs and K, Cs and Rb and Cs, Na and K is particularly preferred. By combinedly containing Cs, the reducing capability is effectively performed, and the luminance is enhanced and the lifetime is prolonged in the organic EL device by the addition into the electron injection region.

In the present invention, an electron injecting layer made of an electrically insulating material or a semiconductor may be further disposed between the cathode and the organic layer. The electron injecting layer enables to effectively prevent a leak of electric current and to improve the electron injection property. As the electrically insulating material, at least one metal compound selected from the group consisting of chalcogenides of alkali metals, chalcogenides of alkaline earth metals, halides of alkali metals and halides of alkaline earth metals is preferable. It is preferable that the electron injecting layer is constituted with the above metal compound since the electron injecting property can be further improved. Preferable examples of the alkali metal chalcogenide include Li₂O, K₂O, Na₂S, Na₂Se and Na₂O. Preferable examples of the alkaline earth metal chalcogenide include CaO, BaO, SrO, BeO, BaS and CaSe. Preferable examples of the alkali metal halide include LiF, NaF, KF, LiCl, KCl and NaCl. Preferable examples of the alkaline earth metal halide include fluorides such as CaF₂, BaF₂, SrF₂, MgF₂ and BeF₂ and halides other than the fluorides.
Examples of the semiconductor constituting the electron transporting layer include oxides, nitrides and oxide nitrides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound for constituting the electron transporting layer is in the form of a crystallite or amorphous insulating thin film. When the electron transporting layer is constituted with the above insulating thin film, a more uniform thin film can be formed and defective pixels such as dark spots can be decreased. Examples of the inorganic compound include chalcogenides of alkali metals, chalcogenides of alkaline earth metals, halides of alkali metals and halides of alkaline earth metals which are described above.

### (VII) Cathode

The cathode is formed from an electrode substance such as metal, alloy, electrically conductive compound or a mixture thereof each having a small work function (4 eV or smaller) to ensure the electron injection into the electron injecting and transporting layer or a light emitting layer. Examples of the electrode substance include sodium, sodium-potassium alloy, magnesium, lithium, magnesium-silver alloy, aluminum / aluminum oxide, aluminum-lithium alloy, indium, rare earth metal, etc.
The cathode is prepared by forming a thin film of the electrode material described above in accordance with a process such as the vapor deposition process and the sputtering process.
When the light emitted from the light emitting layer is taken out of the cathode, it is preferable that the cathode has a transmittance of greater than 10 % to the emitted light.
It is also preferable that the sheet resistivity of the cathode is several hundreds Ω/□ or smaller and the thickness of the cathode is, in general, selected from 10 nm to 1 µm and preferably from 50 to 200 nm.

### (VIII) Insulating layer

In general, an organic EL device tends to form defects in pixels due to leak and short circuit since an electric field is applied to ultra-thin films. To prevent the defects, a layer of an insulating thin film is preferably inserted between the pair of electrodes.
Examples of the material employed for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds can also be employed.

### (IX) Fabrication process of the organic EL device

The organic EL device of the present invention is fabricated, for example, by forming an anode, a light emitting layer, an optional hole injecting and transporting layer, an optional electron injecting and transporting layer, and a cathode in accordance with the process using the materials each being described above. Alternatively, each layer may be formed in a reverse order from the cathode to the anode.
An embodiment of the fabrication of an organic EL device having a construction of anode / hole injecting layer / light emitting layer/electron injecting layer/cathode in this order on a light-transmitting substrate will be described in the following.
First, on a suitable light-emitting substrate, a thin film of an anode substance is formed so as to have a film thickness of 1 µm or thinner, preferably from 10 nm to 200 nm in accordance with a vapor deposition process, a sputtering process, etc. Then, a hole injecting layer is formed on the anode. The hole injecting layer can be formed in accordance with the vacuum vapor deposition process, the spin coating process, the casting process or the LB process, as described above. The vacuum vapor deposition process is preferable because a uniform film can be easily obtained and pinhole is little formed. When the hole injecting layer is formed in accordance with the vacuum vapor deposition process, the conditions are preferably selected from the following ranges: temperature of deposition source: 50 to 450 °C; degree of vacuum: 10⁻⁷ to 10⁻³ Torr; vapor deposition rate: 0.01 to 50 nm/s; temperature of substrate: 50 to 300 °C; and film thickness: 5 nm to 5 µm; although depending on the employed compound (material for hole injecting layer), the crystal structure and the recombination structure.

Subsequently, the light emitting layer is formed on the hole injecting layer by depositing a thin film of the organic light emitting material in accordance with the vacuum vapor deposition process, the sputtering process, the spin coating process or the casting process. The vacuum vapor deposition process is preferable because a uniform film can be easily obtained and pinhole is little formed. When the light emitting layer is formed in accordance with the vacuum vapor deposition process, the conditions of the vacuum vapor deposition can be selected in the same ranges as in the deposition of the hole injecting layer, although depending on the compound to be used.
Next, the electron injecting layer is formed on the light emitting layer formed above. Similarly to the formation of the hole injecting layer and light emitting layer, the electron injecting layer is preferably formed in accordance with the vacuum vapor deposition process, because a uniform film is required. The conditions of the vacuum vapor deposition can be selected from the same ranges as in the formation of the hole injecting layer and light emitting layer.
Although depending on which region of a light emitting region or a hole transporting region the aromatic amine derivative of the present invention is contained in, it may be commonly vapor deposited together with other materials. In addition, when the spin coating process is employed, it may be contained therein by blending it with other materials.
Finally, the cathode is formed on the electron injecting layer, to obtain an organic EL device.
The cathode is made of a metal and can be formed in accordance with the vacuum vapor deposition process or the sputtering process.
However, the vacuum vapor deposition process is preferably employed in order to prevent the underlying organic layers from being damaged during the formation of the film.
In the above fabrication of the organic EL device, the layers from the anode to the cathode are successively formed preferably after a single evacuation operation.

The process for forming the layers in the organic EL device of the present invention is not particularly limited. A conventional process such as the vacuum vapor deposition process and the spin coating process or so can be employed. The organic thin film layer containing the compound of the formula (1) included in the organic EL device of the present invention can be formed in accordance with the vacuum vapor deposition process, the molecular beam epitaxy process (the MBE process) or a known method by dissolving the compound in a solvent and coating the solution according to the dipping process, the spin coating process, the casting process, the bar coating process or the roller coating process.
The thickness of each layer in the organic thin film layer in the organic EL device of the present invention is not particularly limited. In general, an excessively thin layer tends to have defects such as pinholes, and an excessively thick layer requires a high applied voltage and results in decreasing the efficiency. Therefore, the thickness is preferably from several nm to 1 µm.
The organic EL device emits light when a direct voltage of 5 to 40 V is applied with the anode being + terminal and the cathode being - terminal. In the reverse polarity, no electric current flows and no light is emitted upon the application of voltage. When an alternating voltage is applied, the uniform light emission is observed only in the polarity where the anode is + and the cathode is -. The wave shape of alternating voltage is not limited.

The process for forming the layers in the organic EL device of the present invention is not particularly limited. A conventional process such as the vacuum vapor deposition process and the spin coating process or so can be employed. The organic thin film layer containing the compound of the formula (1) included in the organic EL device of the present invention can be formed in accordance with the vacuum vapor deposition process, the molecular beam epitaxy process (the MBE process) or a known method by dissolving the compound in a solvent and coating the solution according to the dipping process, the spin coating process, the casting process, the bar coating process or the roller coating process.
The thickness of each layer in the organic thin film layer in the organic EL device of the present invention is not particularly limited. In general, an excessively thin layer tends to have defects such as pinholes, and an excessively thick layer requires a high applied-voltage and results in decreasing the efficiency. Therefore, the thickness is preferably from several nm to 1 µm.
The organic EL device emits light when a direct voltage of 5 to 40 V is applied with the anode being + terminal and the cathode being - terminal. In the reverse polarity, no electric current flows and no light is emitted upon the application of voltage. When an alternating voltage is applied, the uniform light emission is observed only in the polarity where the anode is + and the cathode is -. The wave shape of alternating voltage is not limited.

The aromatic amine compound of the present invention has a favorable solubility to organic solvents because of its aromatic amino group. Accordingly, the compound is favorably used for a fabrication of the organic EL device by means of a wet process, and even in a case where the compound of the present invention has too high molecular weight to easily form a thin film by vacuum deposition method, it is easy to form the thin film by means of any application method such as the dipping process, the spin coating process, the casting process, the bar coating process, the roller coating process, etc.
The material for forming luminous coated film of the present invention essentially consists of an organic solvent solution containing the aromatic amine compound. The material for forming a luminous coated film is defined as a material for providing an organic compound layer relating to light emission, specifically a light emitting layer, hole injecting (transporting) layer, electron injecting (transporting) layer, and so on by means of forming a coated film in the organic EL device.

Examples of the organic solvent used for dissolving the aromatic amine compound of the present invention include non-halogen-based solvent and halogen-based solvent.
Examples of the non-halogen-based solvent include ether-based solvent such as dibutylether, tetrahydrofuran, dioxane, anisole, and so on; alcohol-based solvent such as methanol or ethanol, propanol, butanol, pentanol, hexanol, cyclohexanol, methyl cellosolve, ethyl cellosolve, ethylene glycol and so on; aromatic solvent such as benzene, toluene, xylene, ethyl benzene and so on; hydrocarbon-based solvent such as hexane, octane, decane and so on; ester-based solvent such as ethyl acetate, butyl acetate, amyl acetate; etc. Aromatic solvent is preferable among those non-halogen-based solvent.
Examples of the halogen-based solvent include dichloromethane, dichloroethane, chloroform, carbon tetrachloride, tetrachloroethane, trichloroethane, chlorobenzene, dichlorobenzene, chlorotoluene, etc.
Further, the solvent may be used alone, or in combination of two or more kind thereof. Additionally, the employable solvent is not limited to the above examples.
Still further, a dopant may be optionally dissolved in advance, into the solution of the material for forming the luminescent-coated film of the present invention. The amine compound shown by the foregoing general formulae (I) and (II) may be employable as the dopant. Moreover, other various additives may be dissolved in advance, if necessary.

### EXAMPLES

The present invention will be described in further detail with reference to Examples, which does not limit the scope of this invention.

### Synthesis 1 (Synthesis of Compound 1)

The route for synthesis of Compound 1 is shown in the following:

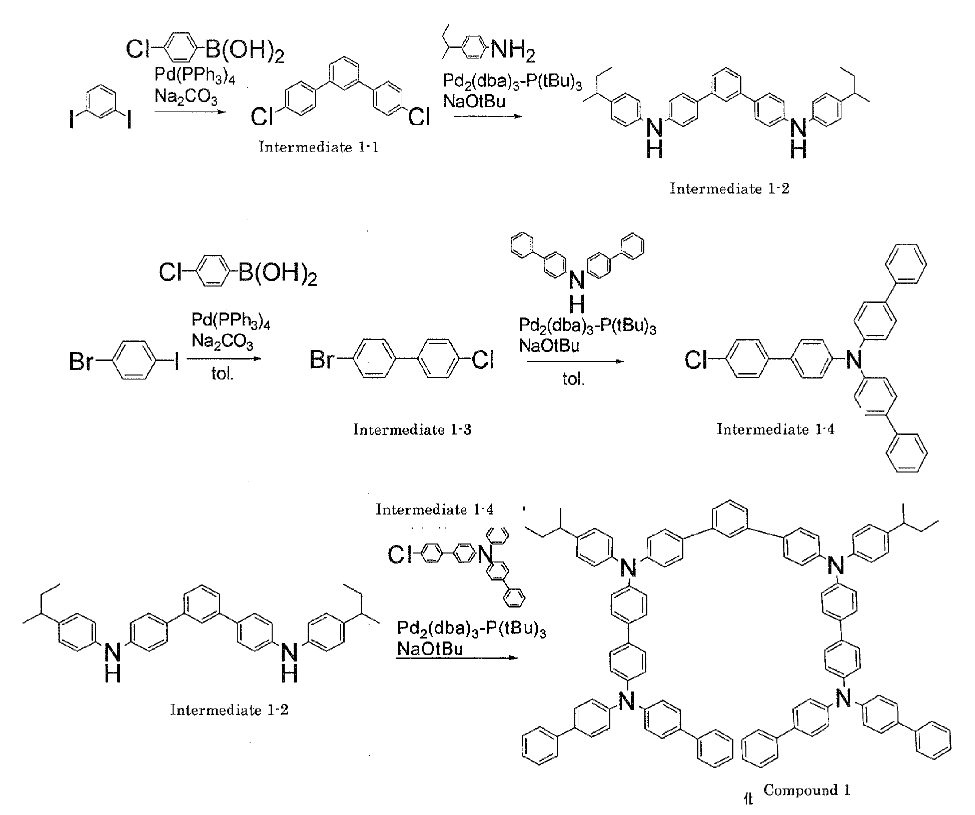

### (1-1) Synthesis of Intermediate 1-1

Under an atmospheric argon gas, 1,3-diiodobenzene (5.0 g, 15 mmol), 4-chlorophenyl boronic acid (5.7 g, 36 mmol), tetrakis(triphenylphosphino)palladium (0.87 g, 0.75 mmol) and 2 mol/l of sodium carbonate aqueous solution (45 ml, 90 mmol) were added into toluene (90 ml) and the resultant solution was stirred at 80 °C for 7 h. An organic layer was separated, and after washing with a saturated sodium chloride aqueous solution, it was dried over anhydrous magnesium sulfate. The dried organic layer was condensed by means of a rotary evaporator and the resultant residue was purified through silicagel column chromatography (eluent: methylene chloride / hexane= 1/3) to obtain white powder (2.7 g, yield: 60 %).

### (1-2) Synthesis of Intermediate 1-2

Under an atmospheric argon gas, Intermediate 1-1 (1.0 g, 3.3 mmol), 4-s-butylaniline (1.2 g, 8.3 mmol), tris(dibenzylideneacetone)dipalladium (60 mg, 66 µmol), sodium t-butoxide (0.95 g, 9.9 mmol) and tri-t-butylphosphine (20 mg, 0.1 mmol) were added into anhydrous toluene (10 ml) and the resultant solution was stirred at 80 °C for 8 h. The reacted solution was purified through silicagel column chromatography (eluent: methylene chloride) to obtain pale white solid (1.3 g, yield: 76 %).

### (1-3) Synthesis of Intermediate 1-3

Under an atmospheric argon gas, dissolving 4-chlorobenzene boronic acid (4.5 g, 29 mmol), 4-bromoiodobenzene (7.3 g, 26 mmol), tetrakis(triphenylphospheno)palladium (0.6 g, 0.52 mmol) into toluene (80 ml), and adding 2 mol/l of sodium carbonate aqueous solution (40 ml, 80 mmol), the resultant solution was stirred at 80 °C for 8 h. An organic layer was separated, and after washing with a saturated sodium chloride aqueous solution, it was dried over anhydrous magnesium sulfate. The dried organic layer was vacuum condensed by means of a rotary evaporator and the resultant residue was purified through silicagel column chromatography (eluent: methylene chloride / hexane= 1/4) to obtain white powder (3.1 g, yield: 44 %).

### (1-4) Synthesis of Intermediate 1-4

Under an atmospheric argon gas, Intermediate 1-3 (3.0 g, 11 mmol), N,N-bis(4-biphenylyl)amine (3.2 g, 10 mmol), tris(dibenzylideneacetone)-dipalladium (180 mg, 0.2 mmol), sodium t-butoxide (1.4 g, 15 mmol) and tri-t-butylphosphine (65 mg, 0.32 mmol) were added into anhydrous toluene (10 ml) and the resultant solution was stirred at 40 °C for 8 h. Adding hexane into the reacted solution, solid was precipitated and washed with methanol to obtain pale white solid (4.8 g, yield: 94 %).

### (1-5) Synthesis of Compound 1

Under an atmospheric argon gas, Intermediate 1-2 (0.57 g, 1 mmol), Intermediate 1-4 (1.2 g, 2.4 mmol), tris(dibenzylideneacetone)dipalladium (73 mg, 80 µmol), sodium t-butoxide (0.29 g, 3 mmol), tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.5 g, yield: 34 %).
FD-MS: Calculated value C₁₁₀H₉₀N₄= 1466, Measured value 1466 (M⁺, 100)
Ionization potential: 5.22 eV
Solubility (toluene): soluble in an amount of 5 wt °/ or more

### Synthesis 2 (Synthesis of Compound 2)

The route for synthesis of Compound 2 is shown in the following.

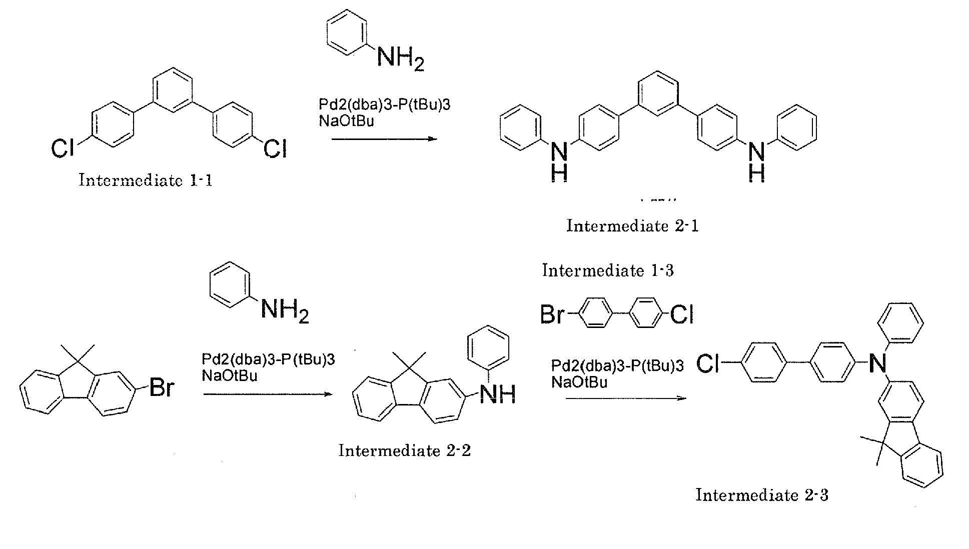

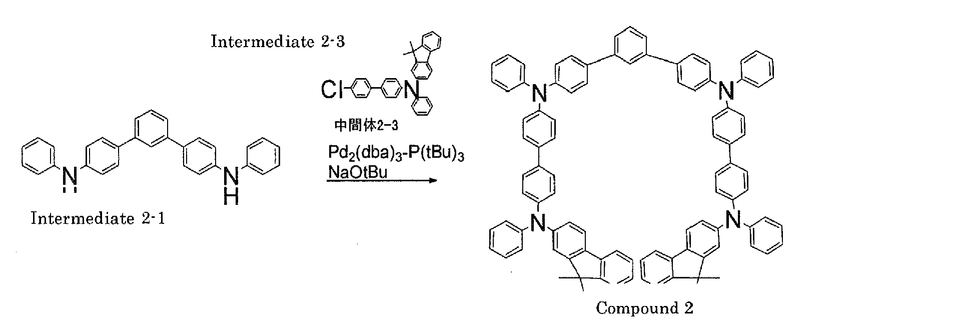

### (2-1) Synthesis of Intermediate 2-1

Under an atmospheric argon gas, Intermediate 1-1 (1.0 g, 3.3 mmol), aniline (0.78 g, 8.3 mmol), tris(dibenzylideneacetone)dipalladium (60 mg, 66 µmol), sodium t-butoxide (0.95 g, 9.9 mmol), tri-t-butylphosphine (20 mg, 0.1 mmol) were added into anhydrous toluene (10 ml) and the resultant solution was stirred at 80 °C for 8 h. The reacted solution was purified through silicagel column chromatography (eluent: methylene chloride) to obtain pale white solid (1.1 g, yield: 81 %).

### (2-2) Synthesis of Intermediate 2-2

Under an atmospheric argon gas, 9,9-dimethyl-2-bromofluorene (1.8 g, 6.6 mmol), aniline (1.56 g, 16 mmol), tris(dibenzylideneacetone)-dipalladium (0.12 g, 0.13 mmol), sodium t-butoxide (1.9 g, 20 mmol) and tri-t-butylphosphine (40 mg, 0.2 mmol) were added into anhydrous toluene (10 ml) and the resultant solution was stirred at 80 °C for 8 h. The reacted solution was purified through silicagel column chromatography (eluent: methylene chloride) to obtain pale white solid (1.5 g, yield: 81 %).

### (2-3) Synthesis of Intermediate 2-3

Under an atmospheric argon gas, Intermediate 1-3 (3.0 g, 11 mmol), Intermediate 2-2 (0.93 g, 10 mmol), tris(dibenzylideneacetone)dipalladium (180 mg, 0.2 mmol), sodium t-butoxide (1.4 g, 15 mmol) and tri-t-butylphosphine (65 mg, 0.32 mmol) were added into anhydrous toluene (20 ml) and the resultant solution was stirred at a room temperature for 8 h. Adding hexane into the reacted solution, solid was precipitated and washed with methanol to obtain Intermediate 2-3 (3.5 g, yield: 75 %).

### (2-4) Synthesis of Compound 2

Under an atmospheric argon gas, Intermediate 2-1 (0.41 g, 1 mmol), Intermediate 2-3 (1.1 g, 2.4 mmol), tris(dibenzylideneacetone)dipalladium (73 mg, 80 µmol), sodium t-butoxide (0.29 g, 3 mmol) and tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.6 g, yield: 47 %).
FD-MS: Calculated value C₉₆H₇₄N₄= 1282, Measured value 1282 (M⁺, 100) Solubility (toluene): soluble in an amount of 1 wt % or more

### Synthesis 3 (Synthesis of Compound 3)

The route for synthesis of Compound 3 is shown in the following.

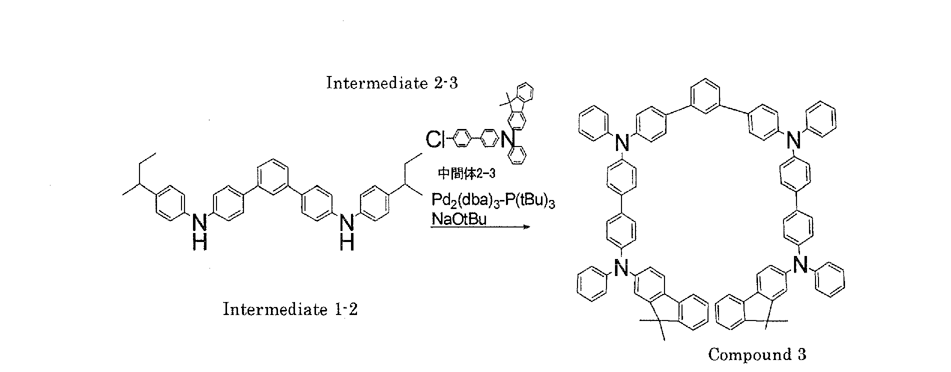

Under an atmospheric argon gas, Intermediate 1-2 (0.53 g, 1 mmol), Intermediate 2-3 (1.1 g, 2.4 mmol), tris(dibenzylideneacetone)dipalladium (73 mg, 80 µmol), sodium t-butoxide (0.29 g, 3 mmol) and tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.25 g, yield: 18 %).
FD-MS: Calculated value C₁₀₄H₉₀N4= 1394, Measured value 1394 (M⁺, 100)
Solubility (toluene): soluble in an amount of 1 wt % or more

### Synthesis 4 (Synthesis of Compound 4)

The route for synthesis of Compound 4 is shown in the following.

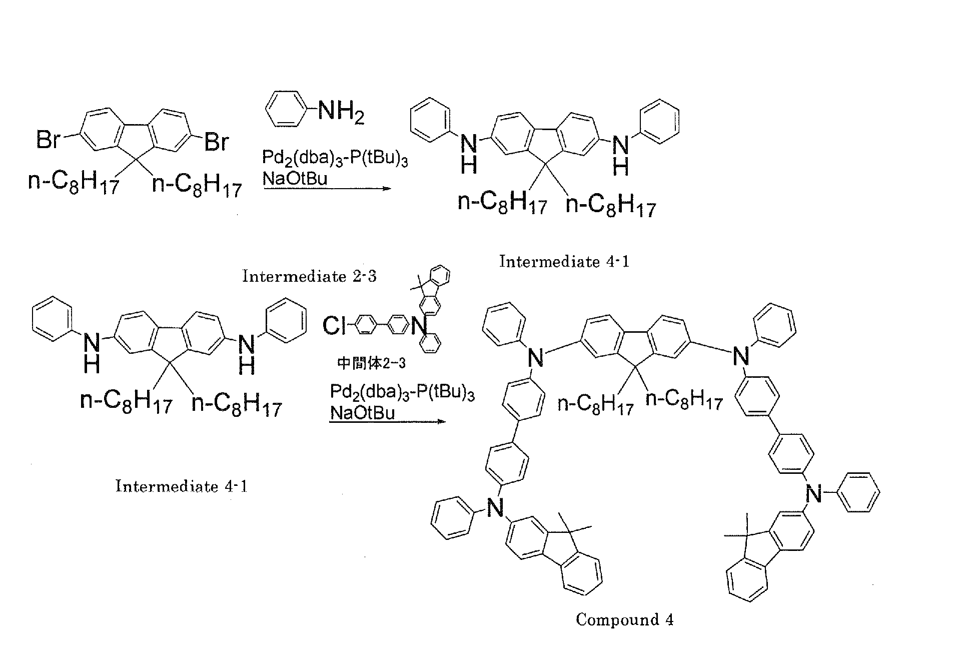

### (4-1) Synthesis of Intermediate 4-1

Under an atmospheric argon gas, 9,9-dioctyl-2,7-dibromofluorene (1.1 g, 3.3 mmol), aniline (0.78 g, 8.3 mmol), tris(dibenzylideneacetone)-dipalladium (60 mg, 66 µmol), sodium t-butoxide (0.95 g, 9.9 mmol) and tri-t-butylphosphine (20 mg, 0.1 mmol) were added into anhydrous toluene (10 ml) and the resultant solution was stirred at 80 °C for 8 h. The reacted solution was purified through silicagel column chromatography (eluent: methylene chloride) to obtain white solid (0.97 g, yield: 85 %).

### (4-2) Synthesis of Compound 4

Under an atmospheric argon gas, Intermediate 4-1 (0.58 g, 1 mmol), Intermediate 2-3 (1.1 g, 2.4 mmol), tris(dibenzylideneacetone)dipalladium (73 mg, 80 µmol), sodium t-butoxide (0.29 g, 3 mmol) and tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.5 g, yield: 35 %).
FD-MS: Calculated value C₁₀₇H₁₀₂N₄= 1442, Measured value 1442 (M⁺, 100)
Solubility (toluene): soluble in an amount of 1 wt % or more

### Synthesis 5 (Synthesis of Compound 5)

The route for synthesis of Compound 5 is shown in the following.

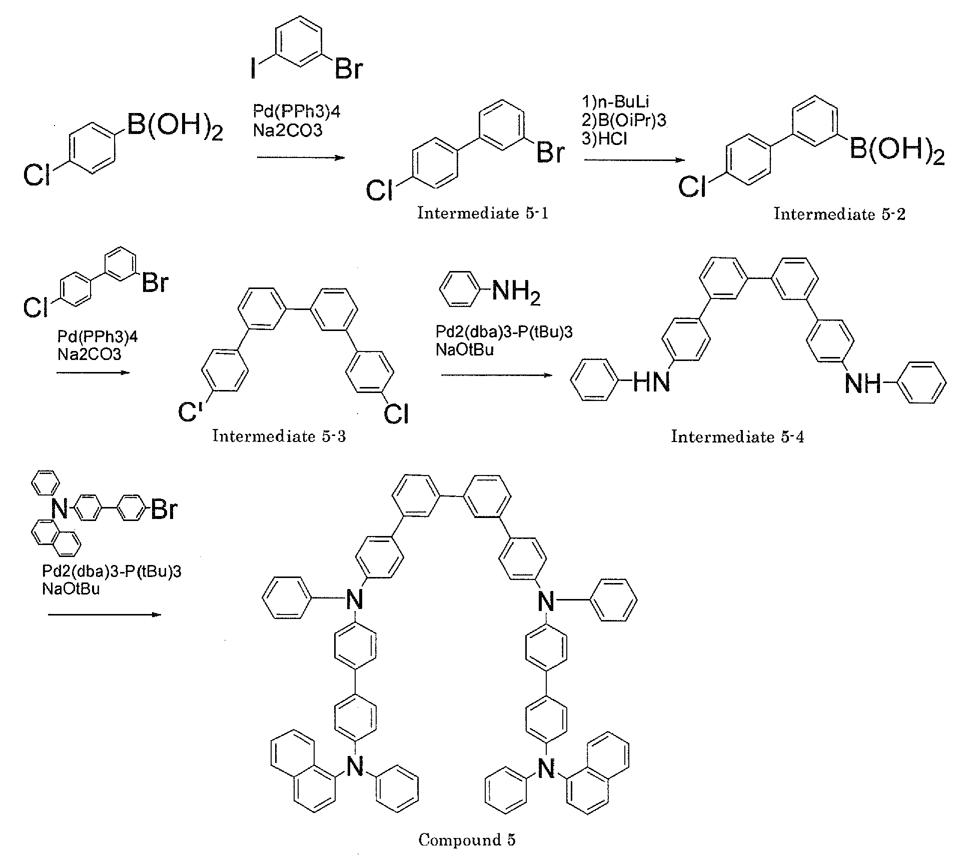

### (5-1) Synthesis of Intermediate 5-1

Under an atmospheric argon gas, 3-bromo-1-iodobenzene (10 g, 36 mmol), 4-chlorophenyl boronic acid (5.7 g, 36 mmol), tetrakis(triphenylphosphino)palladium (0.87 g, 0.75 mmol) and 2 mol/I of sodium carbonate aqueous solution (45 ml, 90 mmol) were added into toluene (90 ml) and the resultant solution was stirred at 80 °C for 7 h. An organic layer was separated, and after washing with a saturated sodium chloride aqueous solution, it was dried over anhydrous magnesium sulfate. The dried organic layer was vacuum condensed by means of a rotary evaporator and the resultant residue was purified through silicagel column chromatography (eluent: methylene chloride / hexane= 1/3) to obtain white powder (7.2 g, yield: 75 %).

### (5-2) Synthesis of Intermediate 5-2

Under an atmospheric argon gas, dissolving Intermediate 5-1 (3 g, 11 mmol) into a liquid mixture of anhydrous tetrahydrofuran (20 ml) and anhydrous toluene (20 ml), and cooled it down to -20 °C. Then, while slowly adding n-butyllithium 1.6M hexane solution (8.1 ml, 13 mmol) the resultant solution was stirred for 1 h. Further cooling down to -60 °C and adding anhydrous tetrahydrofuran solution (10 ml) of boric acid triisopropyl ester (4.1 g, 22 mmol) into the reaction solution, the resultant solution was stirred for 2 h and afterwards, elevating the temperature up to a room temperature, it was stirred for 5 h. Adding 1N hydrochloric acid (30 ml), the resultant solution was stirred for 30 min and an organic layer was separated. The organic layer was vacuum condensed by means of an evaporator and the resultant residue was purified through silicagel column chromatography (hexane / ethylacetate= 1/1) to obtain Intermediate 5-2 (1.6 g, yield: 64 %).

### (5-3) Synthesis of Intermediate 5-3

Under an atmospheric argon gas, Intermediate 5-1 (1.7 g, 6.5 mmol), Intermediate 5-2 (1.5 g, 6.5 mmol), tetrakis(triphenylphosphino)palladium (0.22 g, 0.2 mmol) and 2 mol/l of sodium carbonate aqueous solution (10 ml, 20 mmol) were added into toluene (40 ml) and the resultant solution was stirred at 80 °C for 7 h. An organic layer was separated, and after washing with a saturated sodium chloride aqueous solution, it was dried over anhydrous magnesium sulfate. The organic layer was condensed by means of a rotary evaporator and the resultant residue was purified through silicagel column chromatography (eluent: methylene chloride / hexane= 1/3) to obtain Intermediate 5-3 (1.8 g, yield: 74 %).

### (5-4) Synthesis of Intermediate 5-4

Under an atmospheric argon gas, Intermediate 5-3 (1.2 g, 3.3 mmol), aniline (0.78 g, 8.3 mmol), tris(dibenzylideneacetone)dipailadium (60 mg, 66 µmol), sodium t-butoxide (0.95 g, 9.9 mmol), tri-t-butylphosphine (20 mg, 0.1 mmol) were added into anhydrous toluene (10 ml) and the resultant solution was stirred at 80 °C for 8 h. The reacted solution was purified through silicagel column chromatography (eluent: methylene chloride) to obtain Intermediate 5-4 (1.3 g, yield: 80 %).

### (5-5) Synthesis of Compound 5

Under an atmospheric argon gas, Intermediate 5-4 (0.49 g, 1 mmol), N-(4'-bromobiphenyl-4-yl)-N-(1-naphthyl)-N-phenylamine (1.1 g, 2.4 mmol), tris(dibenzylideneacetone)dipalladium (73 mg, 80 µmol) , sodium t-butoxide (0.29 g, 3 mmol) and tri t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.58 g, yield: 47 %). FD-MS: Calculated value C₉₂H₆₆N₄= 1226, Measured value 1226 (M⁺, 100) Solubility (toluene): soluble in an amount of 1 wt % or more

### Synthesis 6 (Synthesis of Compound 6)

The route for synthesis of Compound 6 is shown in the following.

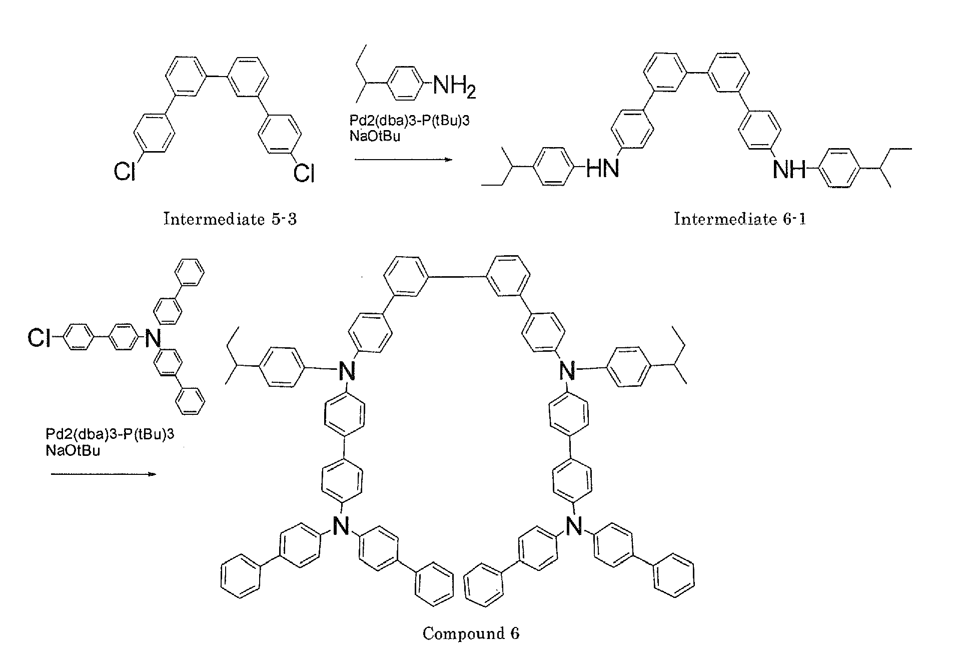

### (6-1) Synthesis of Intermediate 6-1

Under an atmospheric argon gas, Intermediate 5-3 (1.2 g, 3.3 mmol), 4-s-butyl aniline (1.2 g, 8.3 mmol), tris(dibenzylideneacetone)dipalladium (60 mg, 66 µmol), sodium t-butoxide (0.95g, 9.9mmol) and tri-t-butylphosphine (20 mg, 0.1 mmol) were added into anhydrous toluene (10 ml) and the resultant solution was stirred at 80 °C for 8 h. The reacted solution was purified through silicagel column chromatography (eluent: methylene chloride) to obtain pale white solid (1.5 g, yield: 76 %).

### (6-2) Synthesis of Compound 6

Under an atmospheric argon gas, Intermediate 6-1 (0.6 g, 1 mmol), Intermediate 1-4 (1.2 g, 2.4 mmol), tris(dibenzylideneacetone)dipalladium (73 mg, 80 µmol), sodium t-butoxide (0.29 g, 3 mmol), tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.45 g, yield: 29 %).

### Synthesis 7 (Synthesis of Compound 7)

The route for synthesis of Compound 7 is shown in the following.

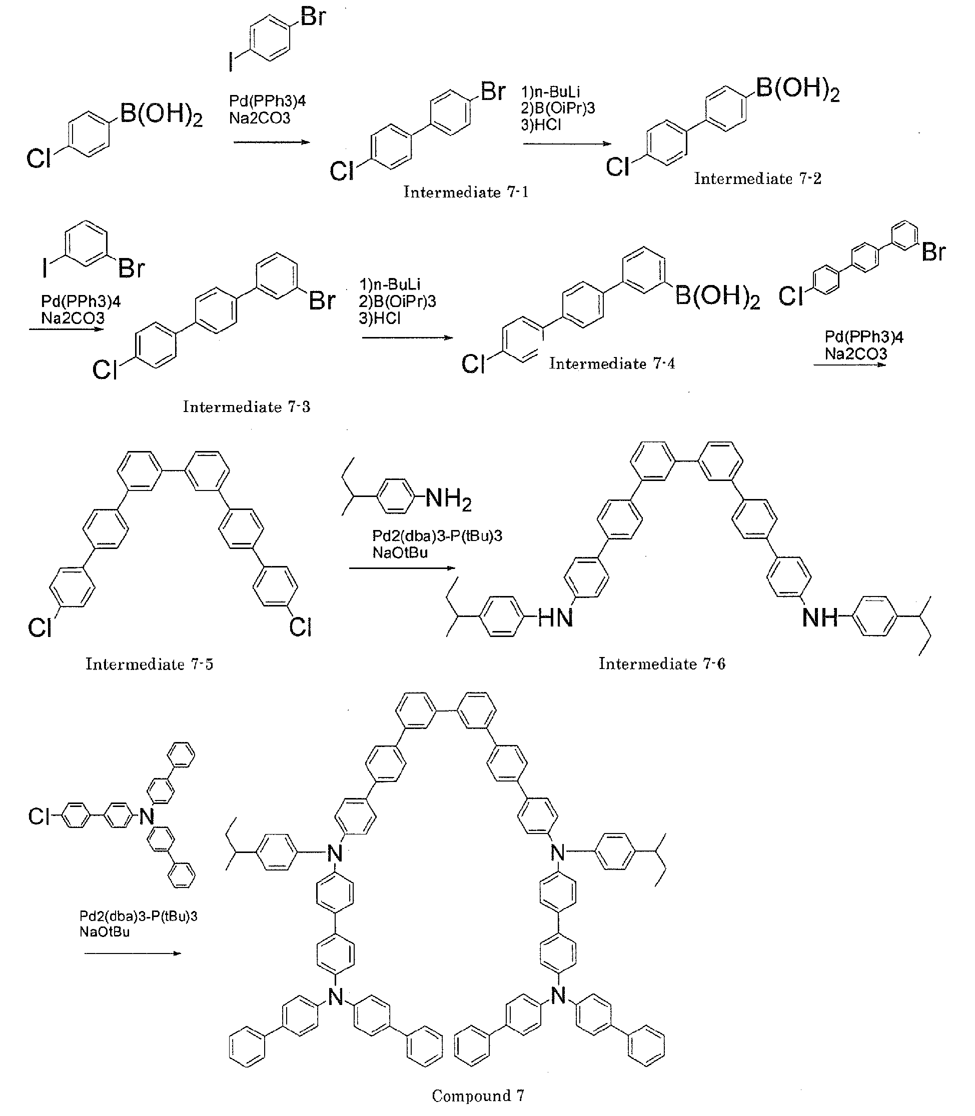

### (7-1) Synthesis of Intermediate 7-1

Under an atmospheric argon gas, 4-bromo-1-iodobenzene (10 g, 36 mmol), 4-chlorophenyl boronic acid (5.7 g, 36 mmol), tetrakis(triphenylphosphino)palladium (0.87 g, 0.75 mmol) and 2 mol/l of sodium carbonate aqueous solution (45 ml, 90 mmol) were added into toluene (90 ml) and the resultant solution was stirred at 80 °C for 7 h. An organic layer was separated, and after washing with a saturated sodium chloride aqueous solution, it was dried over anhydrous magnesium sulfate. The dried organic layer was condensed by means of a rotary evaporator and the resultant residue was purified through silicagel column chromatography (eluent: methylene chloride / hexane= 1/3) to obtain white powder (7.7 g, yield: 81 %).

### (7-2) Synthesis of Intermediate 7-2

Under an atmospheric argon gas, dissolving Intermediate 7-1 (3 g, 11 mmol) into a liquid mixture of anhydrous tetrahydrofuran (20 ml) and anhydrous toluene (20 ml), and cooled it down to -20 °C. Then, while slowly adding n-butyllithium 1.6M hexane solution (8.1 ml, 13 mmol) the resultant solution was stirred for 1 h. Further cooling down to -60 °C and adding anhydrous tetrahydrofuran solution (10 ml) of boric acid triisopropyl ester (4.1 g, 22 mmol) into the reacted solution, the resultant solution was stirred for 2 h and afterwards, elevating the temperature up to a room temperature, it was stirred for 5 h. Adding 1N hydrochloric acid (30 ml), the resultant solution was stirred for 30 min and an organic layer was separated. The organic layer was vacuum condensed by means of an evaporator and the resultant residue was purified through silicagel column chromatography (hexane / ethylacetate= 1/1) to obtain Intermediate 7-2 (1.8 g, yield: 70 %).

### (7-3) Synthesis of Intermediate 7-3

Under an atmospheric argon gas, 3-bromo-1-iodobenzene (1.4 g, 5 mmol), Intermediate 7-2 (1.2 g, 5 mmol), tetrakis(triphenylphosphino)-palladium (0.17 g, 0.15 mmol) and 2 mol/l of sodium carbonate aqueous solution (7.55 ml, 15 mmol) were added into toluene (20 ml) and the resultant solution was stirred at 80 °C for 7 h. An organic layer was separated, and after washing with a saturated sodium chloride aqueous solution, it was dried over anhydrous magnesium sulfate. The organic layer was vacuum condensed by means of a rotary evaporator and the resultant residue was purified through silicagel column chromatography (eluent: methylene chloride / hexane= 1/3) to obtain white powder (1.2 g, yield: 70%).

### (7-4) Synthesis of Intermediate 7-4

Under an atmospheric argon gas, dissolving Intermediate 7-3 (1.2 g, 3.5 mmol) into a liquid mixture of anhydrous tetrahydrofuran (10 ml) and anhydrous toluene (10 ml), and cooled it down to -20 °C. Then, while slowly adding n-butyllithium 1.6M hexane solution (2.6 ml, 4.2 mmol) the resultant solution was stirred for 1 h. Further cooling down to -60 °C and adding anhydrous tetrahydrofuran solution (10 ml) of boric acid triisopropyl ester (1.3 g, 7 mmol) into the reacted solution, the resultant solution was stirred for 2 h and afterwards, elevating the temperature up to a room temperature, it was stirred for 5 h. Adding 1N hydrochloric acid (30 ml), the resultant solution was stirred for 30 min and an organic layer was separated. The organic layer was vacuum condensed by means of an evaporator and the resultant residue was purified through silicagel column chromatography (hexane / ethylacetate= 1/1) to obtain Intermediate 7-4 (0.6 g, yield: 56 %).

### (7-5) Synthesis of Intermediate 7-5

Under an atmospheric argon gas, Intermediate 7-3 (0.66 g, 1.9 mmol), Intermediate 7-4 (0.6 g, 1.9 mmol), tetrakis(triphenylphosphino)palladium (0.07 g, 0.06 mmol) and 2 mol/l of sodium carbonate aqueous solution (2.9 ml, 5.7 mmol) were added into toluene (10 ml) and the resultant solution was stirred at 80 °C for 7 h. An organic layer was separated, and after washing with a saturated sodium chloride aqueous solution, it was dried over anhydrous magnesium sulfate. The organic layer was condensed by means of a rotary evaporator and the resultant residue was purified through silicagel column chromatography (eluent: methylene chloride / hexane= 1/3) to obtain white powder (0.68 g, yield: 68 %).

### (7-6) Synthesis of Intermediate 7-6

Under an atmospheric argon gas, intermediate 7-5 (1.7 g, 3.3 mmol), 4-s-butylaniline (1.2 g, 8.3 mmol), tris(dibenzylideneacetone)dipalladium (60 mg, 66 µmol), sodium t-butoxide (0.95 g, 9.9 mmol), tri-t-butylphosphine (20 mg, 0.1 mmol)) were added into anhydrous toluene (10 ml) and the resultant solution was stirred at 80 °C for 8 h. The reacted solution was purified through silicagel column chromatography (eluent: methylene chloride) to obtain pale white solid (1.5 g, yield: 60 %).

### (7-7) Synthesis of Compound 7

Under an atmospheric argon gas, Intermediate 7-6 (0.75 g, 1 mmol), Intermediate 1-4 (1.2 g, 2.4 mmol), tris(dibenzylideneacetone)dipalladium (73 mg, 80 µmol), sodium t-butoxide (0.29 g, 3 mmol) and tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.54 g, yield: 32 %).
FD-MS: Calculated value C₁₂₈H₁₀₂N₄=1694, Measured value 1694 (M⁺, 100)
Solubility (toluene): soluble in an amount of 1 wt % or more

### Synthesis 8 (Synthesis of Compound 8)

The route for synthesis of Compound 8 is shown in the following.

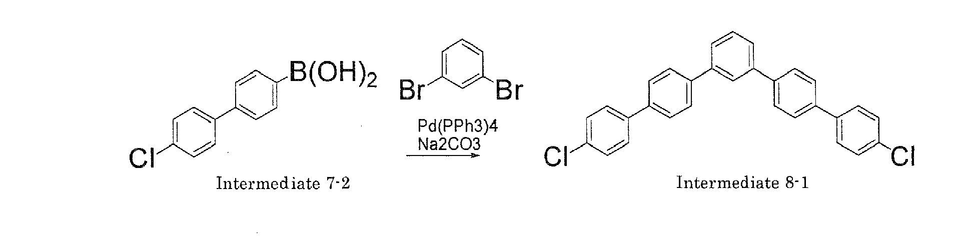

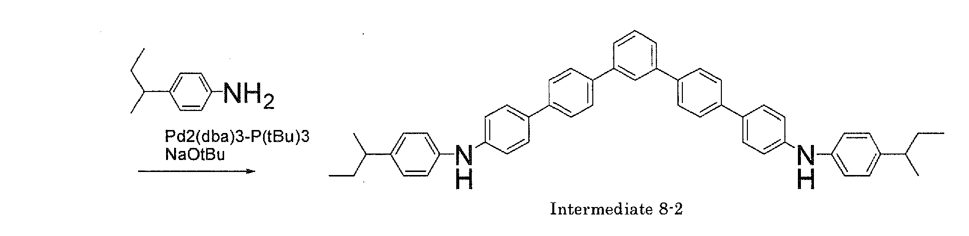

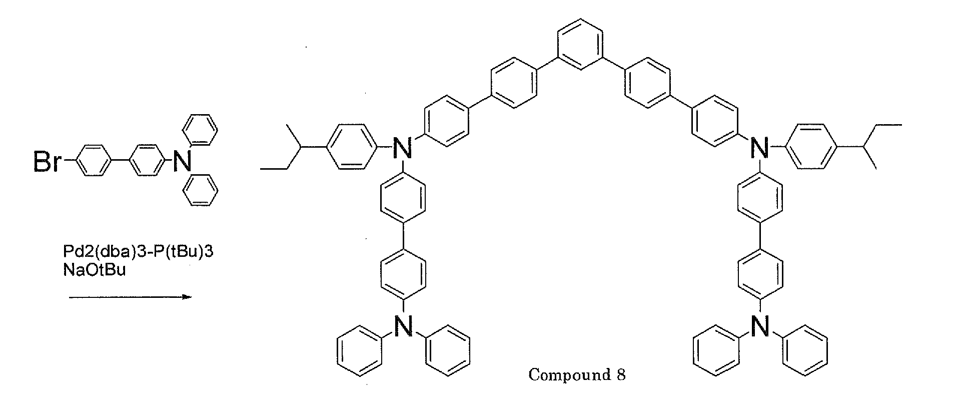

### (8-1) Synthesis of Intermediate 8-1

Under an atmospheric argon gas, Intermediate 7-2 (1.7 g, 13 mmol), 1,3-dibromobenzene (1.5 g, 6.5 mmol), tetrakis(triphenylphosphino) palladium (0.44 g, 0.4 mmol), and 2 mol/l of sodium carbonate aqueous solution (20 ml, 40 mmol) were added into toluene (80 ml) and the resultant solution was stirred at 80 °C for 7 h. An organic layer was separated, and after washing with a saturated sodium chloride aqueous solution, it was dried over anhydrous magnesium sulfate. The organic layer was condensed by means of a rotary evaporator and the resultant residue was purified through silicagel column chromatography (eluent: methylene chloride / hexane= 1/3) to obtain Intermediate 8-1 (2.6 g, yield: 90 %).

### (8-2) Synthesis of Intermediate 8-2

Under an atmospheric argon gas, intermediate 8-1 (1.5 g, 3.3 mmol), 4-s-butylaniline (1.2 g, 8.3 mmol), tris(dibenzylideneacetone)dipalladium (60 mg, 66 µmol), sodium t-butoxide (0.95 g, 9.9 mmol), tri-t-butylphosphine (20 mg, 0.1 mmol)) were added into anhydrous toluene (10 ml) and the resultant solution was stirred at 80 °C for 8 h. The reacted solution was purified through silicagel column chromatography (eluent: methylene chloride) to obtain pale white solid (1.8 g, yield: 79 %).

### (8-3) Synthesis of Compound 8

Under an atmospheric argon gas, Intermediate 8-2 (0.67 g, 1 mmol), Intermediate 1-4 (1.2 g, 2.4 mmol), tris(dibenzylideneacetone)dipalladium (73 mg, 80 µmol), sodium t-butoxide (0.29 g, 3 mmol), tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.50 g, yield: 38 %).
FD-MS: Calculated value C₉₈H₈₂N₄= 1314, Measured value 1314 (M⁺, 100)
Solubility (toluene): soluble in an amount of 1 wt % or more

### Synthesis Example 9 (Synthesis of Compound 9)

The route for synthesis of Compound 9 is shown in the following.

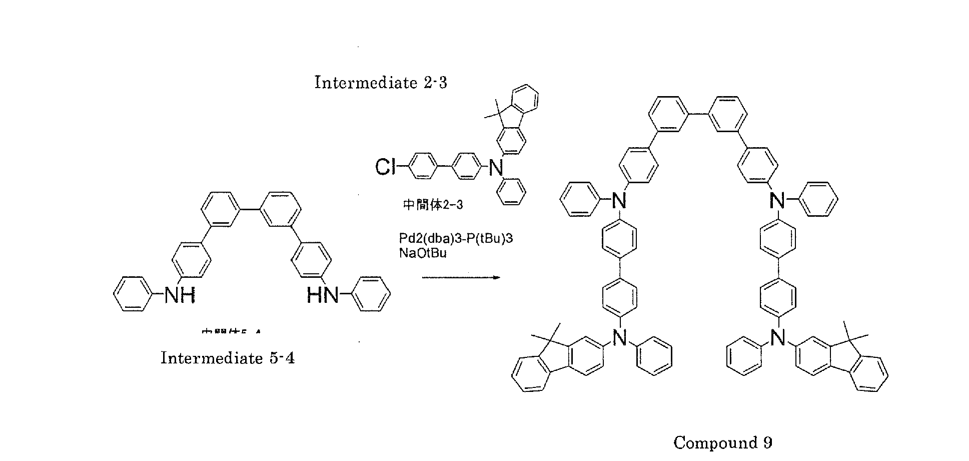

Under an atmospheric argon gas, Intermediate 5-4 (0.67 g, 1 mmol), Intermediate 2-3 (1.2 g, 2.4 mmol), tris(dibenzylideneacetone)dipalladium (73 mg, 80 µmol), sodium t-butoxide (0.29 g, 3 mmol), tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.33 g, yield: 24 %) .
FD-MS: Calculated value C₉₈H₈₂N₄= 1358, Measured value 1358 (M⁺, 100)
Solubility (toluene): soluble in an amount of 1 wt % or more

### Example 1 (Fabrication of an organic EL device)

A glass substrate (manufactured by GEOMATEC Company) of 25 mmX75 mm×1.1 mm thickness having an ITO transparent electrode was cleaned by application of ultrasonic wave in isopropyl alcohol for 5 min and then by exposure to ozone generated by ultraviolet light for 30 min. On the substrate, a film of polyethylenedihydroxythiophene/polystyrene sulfonate (hereinafter PEDOT/PSS) for a hole injecting layer having a thickness of 40 nm was formed in accordance with a spin coating process. Subsequently, using a solution prepared by dissolving the foregoing Compound 1 into toluene (1 % by mass), a hole transporting layer was formed over the PEDOT/PSS by the spin coating process. The thickness was 50 nm. On the film formed above, Compound A below was vacuum vapor deposited thereby forming another film. The film thickness was 40 nm. At the same time, the following styrylamine Compound B below as a light emitting molecule was deposited with a weight ratio of A : B = 40 : 2. The formed film worked as a light emitting layer. Subsequently, a film of tris(8-quinolinolato)aluminum (hereinafter Alq) having a thickness of 10 nm was formed. The formed film of Alq worked as the electron transporting layer. Thereafter, Li (the source of lithium: manufactured by SAES GETTERS Company) as a reductive dopant and Alq were binary vapor deposited and an Alq : Li film was formed as the electron injecting layer (cathode). On the Alq : Li film, metal aluminum was vapor deposited to form a metal cathode and an organic EL device was fabricated. The device emitted blue light. Emission efficiency at 10 mA/cm² and half lifetime of luminance when the device emitted light with an initial luminance of 1000 cd/m² are described in Table 1.

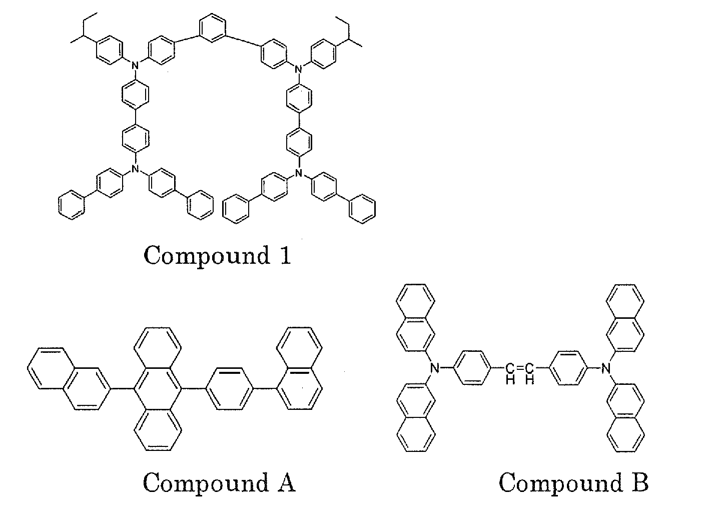

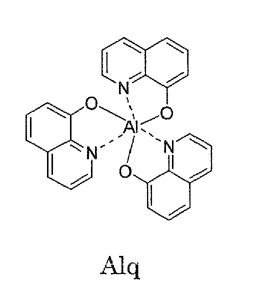

### Example 2

An organic EL device was fabricated in accordance with the same procedures as those conducted in Example 1 except that Compound 1 was replaced with Compound 2 below. The results are shown in Table 1.

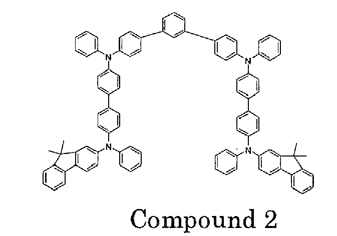

### Example 3

An organic EL device was fabricated in accordance with the same procedures as those conducted in Example 1 except that Compound 1 was replaced with Compound 3 below. The results are shown in Table 1.

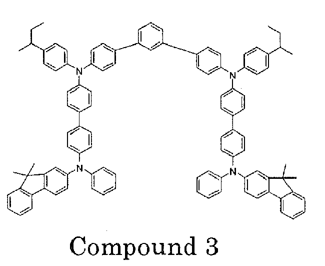

### Example 4

An organic EL device was fabricated in accordance with the same procedures as those conducted in Example 1 except that Compound 1 was replaced with Compound 4 below. The results are shown in Table 1.

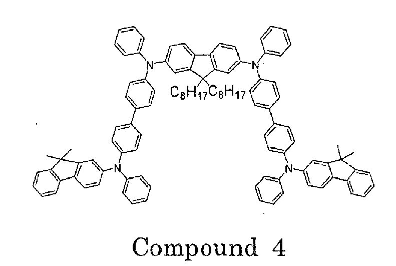

### Example 5

An organic EL device was fabricated in accordance with the same procedures as those conducted in Example 1 except that Compound 1 was replaced with Compound 5 below. The results are shown in Table 1.

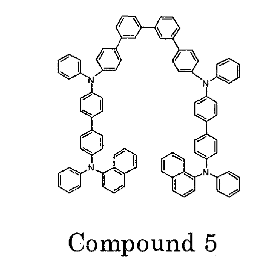

### Example 6

An organic EL device was fabricated in accordance with the same procedures as those conducted in Example 1 except that Compound 1 was replaced with Compound 6 below. The results are shown in Table 1.

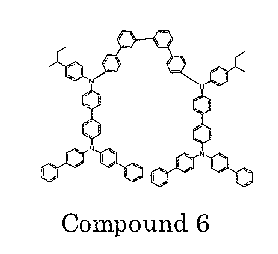

### Example 7

An organic EL device was fabricated in accordance with the same procedures as those conducted in Example 1 except that Compound 1 was replaced with Compound 7 below. The results are shown in Table 1.

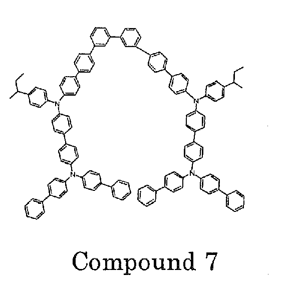

### Example 8

An organic EL device was fabricated in accordance with the same procedures as those conducted in Example 1 except that Compound 1 was replaced with Compound 8 below. The results are shown in Table 1.

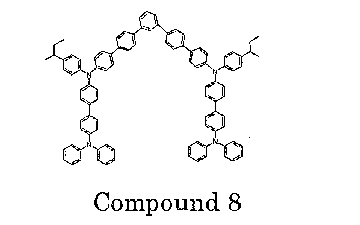

### Example 9

An organic EL device was fabricated in accordance with the same procedures as those conducted in Example 1 except that Compound 1 was replaced with Compound 9 below. The results are shown in Table 1.

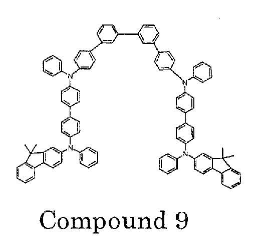

### Comparative Example 1

A preparation of 1 % by mass toluene solution was tried employing Compound C below instead of Compound 1 in accordance with the same procedures as those conducted in Example 1. However, Compound C did not dissolve in toluene and became muddy white.

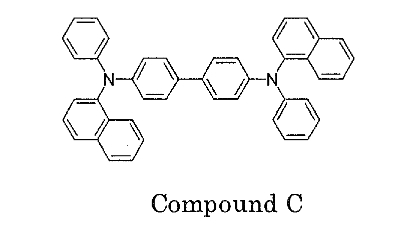

### Comparative Example 2

A preparation of 1 % by mass toluene solution was tried employing Compound D below instead of Compound 1 in accordance with the same procedures as those conducted in Example 1. However, Compound D did not dissolve in toluene and became muddy white.

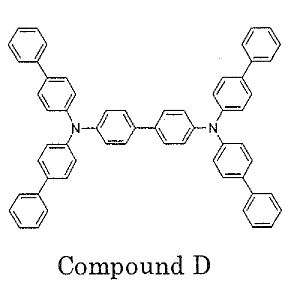

**Table 1**

| Examples | 1 | 2 | 3 | 4 | 5 | 6 | 7 | 8 | 9 |
|---|---|---|---|---|---|---|---|---|---|
| Emission efficiency (cd/A) | 8.6 | 7.9 | 8.1 | 8.6 | 7.8 | 8.2 | 8.3 | 8.1 | 8.0 |
| Half lifetime (h) | 1000 | 1250 | 1100 | 1050 | 1200 | 1050 | 900 | 950 | 1000 |

### Example 10 (Fabrication of an organic EL device)

A glass substrate (manufactured by GEOMATEC Company) of 25 mmX75 mm×1.1 mm thickness having an ITO transparent electrode was cleaned by application of ultrasonic wave in isopropyl alcohol for 5 min and then by exposure to ozone generated by ultraviolet light for 30 min. On the substrate, a film of polyethylenedihydroxythiophene/polystyrene sulfonate (PEDOT/PSS) for a hole injecting layer having a thickness of 100 nm was formed in accordance with a spin coating process. Subsequently, using a solution prepared by dissolving the foregoing Compound 1 into toluene (0.6 % by mass), a layer with a thickness of 20 nm was formed by a spin coating process, dried at 170 °C for 30 min.
Subsequently, using a solution prepared by dissolving Compound E : Compound F below (1 % by mass solution in toluene / isopropanol, E : F = 20 : 2 (mass ratio), (toluene / isopropanol =1/2 (mass ratio)), a light emitting layer was formed in accordance with a spin coating process. The film thickness was 40 nm. On the film formed above, a film of Alq having a thickness 10 nm was formed. The formed film of Alq worked as the electron transporting layer. Thereafter, Li (the source of lithium: manufactured by SAES GETTERS Company) as a reductive dopant and Alq were binary vapor deposited and an Alq : Li film was formed as the electron injecting layer (cathode). On the Alq : Li film, metal aluminum was vapor deposited to form a metal cathode and an organic EL device was fabricated. The device emitted blue light, and voltage at a luminance of 100 cd/m² and emission efficiency are described in Table 1.

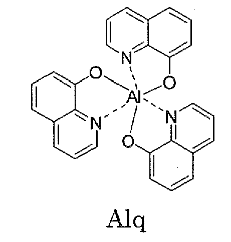

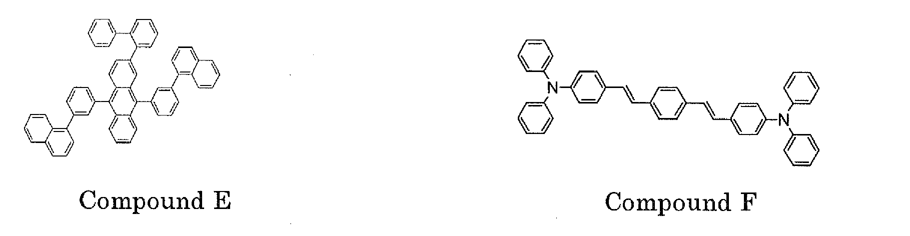

### Synthesis 10 (Synthesis of Compound 10)

The route for synthesis of Compound 10 is shown in the following.

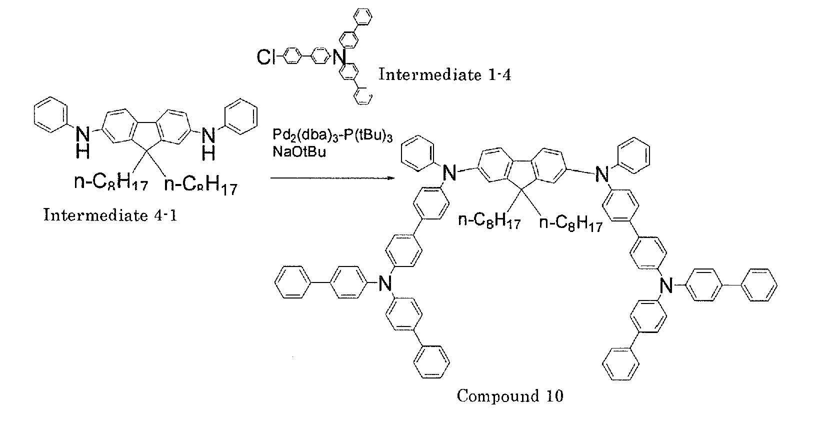

Under an atmospheric argon gas, Intermediate 4-1 (0.57 g, 1 mmol), Intermediate 1-4 (1.2 g, 2.4 mmol), tris(dibenzylideneacetone)dipalladium (73 mg, 80 µmol), sodium t-butoxide (0.29 g, 3 mmol), tri-t-butylphosphine (26 mg, 0.13 mmol) were added into anhydrous toluene (20 ml), and the resultant solution was stirred at 80 °C for 8 h. The reacted solution was purified through silicagel column chromatography (eluent: methylene chloride), and successively through silicagel column chromatography (eluent: methylene chloride / hexane= 1/3) to obtain yellow solid (0.35 g, yield: 23 %).
FD-MS: Calculated value C₁₁₃H₁₀₂N₄= 1514, Measured value 1514 (M⁺, 100)
Ionization potential: 5.14 eV
Solubility (toluene): soluble in an amount of 5 wt % or more

### Example 11

An organic EL device was fabricated in accordance with the same procedures as those conducted in Example 10 except that Compound 1 was replaced with Compound 10 below. The results are shown in Table 2.

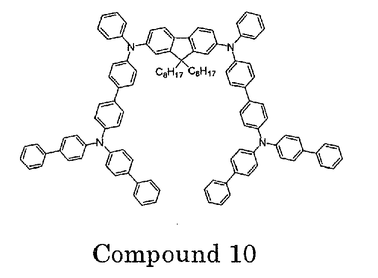

### Example 12

An organic EL device was fabricated in accordance with the same procedures as those conducted in Example 10 except that Compound 1 was replaced with Compound 6. The results are shown in Table 2.

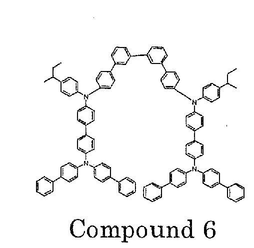

### Example 13

An organic EL device was fabricated in accordance with the same procedures as those conducted in Example 10 except that Compound 1 was replaced with Compound 3. The results are shown in Table 2.

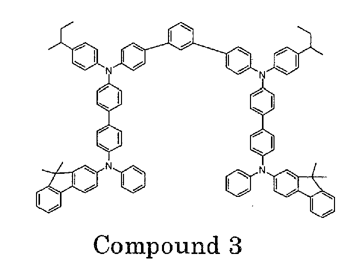

### Comparative Example 3

An organic EL device was fabricated in accordance with the same procedures as those conducted in Example 10 except that Compound 1 was replaced with Compound G below. The results are shown in Table 2.

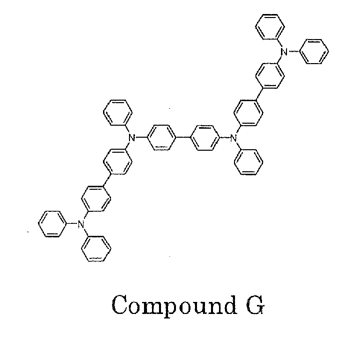

**Table 2**

| | Example 10 | Example 11 | Example 12 | Example 13 | Comparative Example 3 |
|---|---|---|---|---|---|
| Voltage (V) | 5.5 | 5.5 | 5.5 | 5.5 | 5.5 |
| Emission efficiency (cd/A) | 3.5 | 2.9 | 2.8 | 2.9 | 1.5 |

Because the compound of the present technology has an appropriate solubility and solvent resistance of film, the light emitting layer formed by coating revealed an enhanced emission efficiency. On the other hand, as shown in Comparative Example 3, an employment of Compound G revealed inferior emission efficiency to the present technology. Considering about the reason, a large-scale collapse of carrier balance induced from thinning of the film thickness as a result that the hole transporting layer is cut down by solvent during formation of the light emitting layer while coating is estimated.
In conclusion, it is understood that the compound of the present technology is superior both in solubility among a solvent and in durability against solvent in a form of thin film to generally known low molecular compound hole transporting material. Therefore, the compound is promising as the hole transporting material capable of lamination by coating the hole transporting layer and the light emitting layer.

### INDUSTRIAL APPLICABILITY

As described in detail above, the aromatic amine compound of the present invention has high solubility, and a laminated coating of the thin-film-forming solution using the compound in a production process of the organic EL device is possible, having film-forming ability by wet process. The organic EL device using the compound reveals various kinds of light emission hue, and a high heat resistance. Particularly, employing the aromatic amine compound of the present invention as a hole injecting and transporting material will provide the device with an enhanced luminance of light emission, an enhanced efficiency of light emission and a prolonged lifetime because of its enhanced hole injecting and transporting property. The organic EL device of the present invention is useful for a planar light emitting member for wall televisions and a light source for a backlight of displays.

## Claims

1. An aromatic amine compound represented by the following general formula (1): 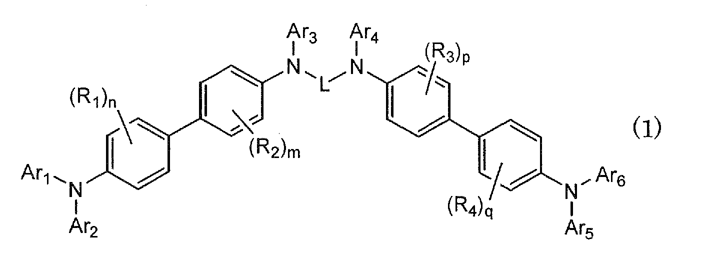 wherein Ar₁ to Ar₆ each independently represents a substituted or unsubstituted aryl group having 6 to 40 ring carbon atoms or a substituted or unsubstituted heteroaryl group having 3 to 40 ring carbon atoms;
each pair of Ar₁ and Ar₂, and Ar₅ and Ar₆ may be bonded each other with single bond, and may form a carbazolyl group;
R₁ to R₄ each independently represents a halogen atom, a carboxyl group, an amino group, a hydroxy group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, or a substituted or unsubstituted alkoxy group having 1 to 40 carbon atoms;
n, m, p and q each represents an integer of 0 to 4;
L is a divalent linking group represented by the following general formula (2) or the following general formula (3):
-(Ar₇)ₐ-(Ar₈)_{b}-(Ar₉)_{c}- (2)
wherein Ar₇ to Ar₉ each represents a divalent group based on the groups represented by the above Ar₁ to Ar₆, a, b and c each represents an integer of 1 to 3; 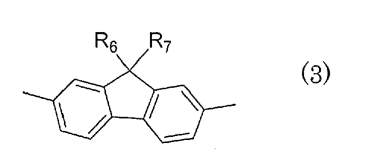 wherein R₆ and R₇ are the same as described above about R₁ to R₄, and a pair of R₆ and R₇ may be bonded each other to form a ring structure.

2. The aromatic amine compound according to claim 1, wherein the aromatic amine compound is used as a material for organic electroluminescence devices.

3. The aromatic amine compound according to claim 1, wherein the aromatic amine compound is used as a hole transporting material for organic electroluminescence devices or a hole injecting material for organic electroluminescence devices.

4. An organic electroluminescence device which comprises one or more organic thin film layers sandwiched between a pair of electrodes consisting of an anode and a cathode, wherein at least one of the organic thin film layers comprises the aromatic amine compound according to claim 1.

5. The organic electroluminescence device according to claim 4, wherein the organic thin film layer comprises at least one of a hole transporting region and a hole injecting region, and wherein the aromatic amine compound is contained in at least one of the hole injecting region and the hole transporting region.

6. The organic electroluminescence device according to claim 5, wherein at least one of the hole transporting region and the hole injecting region contains the aromatic amine compound as its essential component.

7. The organic electroluminescence device according to any one of claims 4 to 6, wherein at least one of the hole transporting region and the hole injecting region essentially contains the aromatic amine compound, and wherein a light emitting layer is formed in accordance with a vacuum vapor deposition process.

8. The organic electroluminescence device according to any one of claims 4 to 6, wherein at least one of the hole transporting region and the hole injecting region essentially comprises the aromatic amine compound, and wherein a light emitting layer is formed in accordance with a wet process.

9. A thin-film-forming solution for an organic electroluminescence, which comprises the aromatic amine compound according to claim 1 in an amount of 0.1 % by weight or more.
